# EUROPÄISCHE PATENTSCHRIFT

(11) **EP 3 020 480 B1**
(45) Veröffentlichungstag und Bekanntmachung des Hinweises auf die Patenterteilung: **02.10.2019**
(21) Anmeldenummer: 14193245.9
(22) Anmeldetag: 14.11.2014
(51) Int. Cl.: B01L 3/00

(54) **FLUIDKANALSYSTEM ZUR UNTERSUCHUNG VON ZELLEN**
FLUID CHANNEL SYSTEM FOR EXAMINING CELLS
CONDUITE DE FLUIDE DESTINÉ À L'EXAMEN DE CELLULES

(43) Veröffentlichungstag der Anmeldung: 18.05.2016
(73) Patentinhaber: ibidi GmbH, 82152 Martinsried (DE)
(72) Erfinder: Zantl, Roman, 85598 Baldham (DE); Dietrich, Miriam, 81373 München (DE)
(74) Vertreter: Grünecker Patent- und Rechtsanwälte PartG mbB

(56) Entgegenhaltungen:
- WO-A1-2009/061392
- WO-A2-03/061585
- US-A1- 2003 156 991
- US-A1- 2013 096 029

## Beschreibung

Die Erfindung betrifft ein Fluidkanalsystem zur Untersuchung von Zellen.

Das Verhalten von Zellen ist abhängig von den Umgebungsbedingungen, wie etwa der Konzentration von Nährstoffen, Salzen, pH-Wert, Temperatur, Wachstumsfaktoren und den Konzentrationen von bestimmten Gasen, wie z.B. Sauerstoff. Weitere wichtige Parameter sind die Dimensionalität, in der sich die Zellen befinden, die Kommunikation mit anderen Zellen, die dazu in unmittelbare Nachbarschaft in definierter Weise angeordnet sein müssen, und die Steifigkeit der Oberflächen bzw. Matrices, mit denen die Zellen interagieren, z.B. über Adhäsion. Die Schaffung von Bedingungen, die ähnlich zu denen im lebenden Organismus sind, ist eine aktuelle Herausforderung der Zellbiologie. Dies dient insbesondere dazu, die Wirksamkeit von Medikamenten oder auch grundlegende wissenschaftliche Fragestellungen unter möglichst relevanten Umgebungsbedingungen, die den in vivo Bedingungen ähnlich sind, zu untersuchen. Hydrogele sind in vielerlei Hinsicht gut geeignet, um die mechanischen und hydrodynamischen Bedingungen in Gewebe zu simulieren.

Aus dem Stand der Technik ist bekannt, Zellen in dreidimensionalen, in Fluidkanälen angeordneten Hydrogelen zu kultivieren, deren Steifigkeit an die der zellspezifischen Umgebungsparameter im Organismus anpassbar sind, unter anderem zur Untersuchung von Zellen, die in Hydrogelen immobilisiert sind.

Beispielsweise wird in Heo et al. "A Microfluidic Bioreactor Based on Hydrogel-Entrapped E.coli: Cell Viability, Lysis, and Intracellular Enzyme Reactions" (Anal. Chem. 2003, 75, 22-26) beschrieben, wie E.coli Zellen in Hydrogel-Mikroflecken, die in mikrofluidischen Systemen fotopolymerisiert sind, eingeschlossen werden. Diese Mikroflecken haben eine Größenordnung von 100 µm bis 500 µm. Kleine Moleküle in einer Lösung, die das Hydrogel umgibt, diffundieren in das Gel und treffen auf Zellen. Ähnliche Systeme sind auch zum Nachweis von Antikörpern bekannt. Beispielsweise zeigt Ikami et al. (Immuno-pillar chip: a new platform for rapid and easy-touse immunoassay, Lab Chip, 2010, 10, 3335-3340) Hydrogel-Säulen, die in einem Mikrokanal durch Fotopolymerisierung ausgebildet werden und in denen anti-CRP Antikörpermoleküle immobilisiert sind.

Der Vorteil der oben beschriebenen Systeme ist, dass die Kanäle selbst sehr einfach ausgebildet sein können. Das Problem ist jedoch, dass gerade bei der Untersuchung von Zellwachstum die gezeigten Hydrogel-Säulen oder Hydrogel-Mikroflecken zu kleine Strukturen sind, um die Gegebenheiten in echtem Gewebe realistisch nachzubilden.

Alternative Kanalsysteme, in denen auch größere Hydrogel-Abschnitte möglich sind, werden beispielsweise in Shin et al. "Microfluidic assay for simultaneous culture of multiple cell types on surfaces or within hydrogels" (Nature Protocols, Vol. 7 No. 7, 2012, 1247-1259) beschrieben. In dieser Veröffentlichung wird beschrieben, wie dreidimensionale Zellkulturen untersucht werden, indem sie in Hydrogel eingebracht werden, das sich in Kammern zwischen zwei Mikrokanälen befindet. Ein ähnlicher Aufbau wird auch in Chung et al. "Cell migration into scaffolds under coculture conditions in a microfluidic platform" (Lab Chip, 2009, 9, 269-275) beschrieben. In beiden Fällen ist der Aufbau relativ kompliziert, weil das flüssige Hydrogel, das durch eigens dafür vorgesehene Einfüllöffnungen in die Kammer eingebracht wird, nicht in die Kanäle fließen darf. Daher muss die Kammer so aufgebaut sein, dass alleine mittels Oberflächenspannung das flüssige Hydrogel in der Kammer gehalten wird. In beiden Fällen werden dafür Säulen verwendet, die in dem Bereich angeordnet sind, in dem das Hydrogel fixiert werden soll.

Wie bereits erwähnt, sind hier auch größere Hydrogel-Abschnitte möglich, das System ist jedoch relativ kompliziert, da für jede Kammer Einfüllöffnungen und Elemente zum Erhöhen der Oberflächenspannung vorgesehen sein müssen.

Ein weiteres alternatives, relativ kompliziert aufgebautes Kanalsystem ist in WO2009/061392 offenbart.

Daher ist eine Aufgabe der Erfindung, ein Fluidkanalsystem zur Untersuchung von Zellen bereitzustellen, das bei einem vereinfachten Aufbau ermöglicht, möglichst realistische Randbedingungen für Zellwachstum bereitzustellen. Diese Aufgabe wird durch den Gegenstand der unabhängigen Patentansprüche gelöst.

Das erfindungsgemäße Fluidkanalsystem zur Untersuchung von Zellen umfasst eine Kammer, die mit mindestens einem fotopolymerisierten Hydrogel und/oder einem polymerisierten fotodepolymerisierbaren Hydrogel zu mindestens 90%, insbesondere zu mindestens 95%, insbesondere vollständig, gefüllt ist, wobei die Kammer über mindestens zwei Öffnungen ausschließlich zu mindestens einem Fluidkanal hin offen ist, wobei der mindestens eine Fluidkanal und die Kammer jeweils in Form eines Hohlraums in einem Substrat ausgebbildet sind und wobei jeder Fluidkanal zwei Öffnungen nach außen aufweist.

Ein weiteres erfindungsgemäßes Fluidkanalsystem zur Untersuchung von Zellen umfasst einen Hohlraum in einem Substrat, wobei in dem Hohlraum mindestens ein fotopolymerisiertes Hydrogel und/oder ein polymerisiertes fotodepolymerisierbares Hydrogel angeordnet ist, wobei das Hydrogel jeweils derart strukturiert ist, dass mindestens ein Fluidkanal und eine zusammenhängende Hydrogelstruktur ausgebildet sind, und wobei die Hydrogelstruktur an zwei getrennten Oberflächenbereichen an den mindestens einen Fluidkanal angrenzt und ausschließlich über den mindestens einen Fluidkanal eine Verbindung nach außen hat.

Die oben genannten erfindungsgemäßen Fluidkanalsysteme haben jeweils den Vorteil, dass ihre Struktur vergleichsweise einfach ist und dennoch realistische Randbedingungen für Zellwachstum bereitgestellt werden.

Insbesondere kann man bei der Verwendung das obengenannten Fluidkanalsystem die Versorgung von Zellen in einem Gewebe im Körper nachstellen, weil in den mindestens einen Fluidkanal eine Flüssigkeit mit Partikeln eingefüllt und über die Öffnungen nach außen ein Druck angelegt werden kann, der dem interstitiellem Druck im Körper ähnelt und das Eindringen der Partikel in das Hydrogel fördert.

Jeder Fluidkanal hat zwei Öffnungen nach außen und mindestens eine Öffnung zu der Kammer. Bei dem Begriff "Öffnungen nach außen" bezieht sich "außen" dabei auf den Bereich außerhalb des Substrats. Die mindestens zwei Öffnungen der Kammer bzw. die zwei getrennten Oberflächenbereiche sind nicht zusammenhängend ausgebildet. Die Öffnungen der Kammer können beispielsweise durch Wandstücke, die beispielsweise im Substrat ausgebildet sein können, getrennt sein.

Sofern anwendbar und nicht anders spezifiziert, sind Merkmale, die im Folgenden nur für die Öffnungen der Kammer beschrieben sind, ebenfalls auf die Oberflächenbereiche anwendbar.

Dass die Kammer ausschließlich zu dem mindestens einen Fluidkanal hin offen ist bedeutet, dass sie keine direkte Verbindung nach außen hat sondern ausschließlich über den mindestens einen Fluidkanal eine Verbindung nach außen hat.

Die Kammer kann über genau eine Öffnung zu einem ersten und genau eine Öffnung zu einem zweiten Fluidkanal hin offen sein oder über genau zwei Öffnungen zu dem genau einen Fluidkanal hin offen sein. Die Kammer kann durch eine unterbrochene Wand, die beispielsweise im Substrat ausgebildet sein kann, zu dem mindestens einen Fluidkanal hin begrenzt sein. Öffnungen der Kammer zu dem mindestens einen Fluidkanal bezeichnen dann die Löcher bzw. Unterbrechungen in der unterbrochenen Wand. Andernfalls wird die Kammer als durch die Fortsetzung der Fluidkanalwand bzw. Fluidkanalwände begrenzt betrachtet, die ebenfalls im Substrat ausgebildet sein können. Öffnungen der Kammer zu dem mindestens einen Fluidkanal bezeichnen dann die Unterbrechung in der Fluidkanalwand bzw. den Fluidkanalwänden.

Der mindestens eine Fluidkanal kann genau einen Fluidkanal umfassen, wobei die Kammer an einem ersten Teilbereich des Fluidkanals und an einem zweiten Teilbereich des Fluidkanals zu dem Fluidkanal hin offen ist. Der mindestens eine Fluidkanal kann genau einen Fluidkanal umfassen, wobei einer der zwei Oberflächenbereiche der Hydrogelstruktur an einen ersten Teilbereich des Fluidkanals angrenzt und der andere der zwei Oberflächenbereiche der Hydrogelstruktur an einen zweiten Teilbereich des Fluidkanals angrenzt.

Das heißt, es kann an jedem Teilbereich des Fluidkanals jeweils eine Öffnung der Kammer bzw. ein Oberflächenbereich angeordnet sein. Insbesondere können zwei der mindestens zwei Öffnungen der Kammer bzw. die beiden Oberflächenbereiche einander gegenüberliegen.

Wenn das Fluidkanalsystem genau einen Fluidkanal umfasst, muss dieser in seinem Verlauf zwischen zwei Öffnungen der Kammer bzw. zwischen zwei Oberflächenbereichen der Hydrogelstruktur so gekrümmt oder abgewinkelt sein, dass die Kammer bzw. die Hydrogelstruktur zumindest teilweise zwischen den zwei Teilbereichen angeordnet ist. Beispielsweise kann der Fluidkanal U-förmig oder V-förmig sein. Die Teilbereiche können beispielsweise in Form von Schenkeln ausgebildet sein.

Der mindestens eine Fluidkanal kann einen ersten Fluidkanal und einen zweiten Fluidkanal umfassen, wobei die mindestens zwei Öffnungen der Kammer eine erste Öffnung zu dem ersten Fluidkanal und eine zweite Öffnung zu dem zweiten Fluidkanal hin umfassen. Der mindestens eine Fluidkanal kann einen ersten Fluidkanal und einen zweiten Fluidkanal umfassen, wobei die mindestens zwei getrennten Oberflächenbereiche einen ersten Oberflächenbereich, der an den ersten Fluidkanal angrenzt, und einen zweiten Oberflächenbereich, der an den zweiten Fluidkanal angrenzt, umfassen. Wenn der mindestens eine Fluidkanal einen ersten Fluidkanal und einen zweiten Fluidkanal umfasst, kann die Kammer bzw. die Hydrogelstruktur ganz oder teilweise zwischen dem ersten und dem zweiten Fluidkanal angeordnet sein.

Das mindestens eine Hydrogel kann die gleiche oder eine andere Form haben als die Kammer. Die Kammer kann auf ihrer gesamten Höhe mit Hydrogel gefüllt sein. Zusätzlich oder alternativ kann die Kammer ihrer gesamten Länge nach mit Hydrogel gefüllt sein. Alternativ oder zusätzlich kann die Kammer auf ihrer gesamten Breite mit Hydrogel gefüllt sein. Das Hydrogel kann sich insbesondere bis hin zu allen im Substrat ausgebildeten Wänden der Kammer erstrecken. Alternativ kann sich das Hydrogel nicht bis hin zu allen im Substrat ausgebildeten Wänden der Kammer erstrecken.

Mindestens 80%, insbesondere mindestens 90%, insbesondere mindestens 95%, insbesondere 100% des Hydrogels in der Kammer kann zusammenhängend ausgebildet sein.

Das Hydrogel in der Kammer bzw. die Hydrogelstruktur kann strukturiert sein, insbesondere so strukturiert, dass in dem Hydrogel bzw. in der Hydrogelstruktur Kanäle ausgebildet sind, insbesondere Kanäle, die den ersten Fluidkanal und den zweiten Fluidkanal bzw. den einen Teilbereich des einzigen Fluidkanals mit dem anderen Teilbereich des einzigen Fluidkanals miteinander verbinden. Solche Kanäle können auch verzweigt sein. Somit kann die Zufuhr von Partikeln aus einer Lösung aus dem bzw. einem der Fluidkanäle in das Hydrogel erhöht werden. Des Weiteren können gegebenenfalls Zellen entlang der Kanäle wachsen. Alternativ oder zusätzlich können in dem Hydrogel in der Kammer bzw. in der Hydrogelstruktur Hohlräume vorhanden sein, die auch miteinander verbunden sein können. Für manche Versuchsaufbauten kann es von Vorteil sein, wenn sich Flüssigkeit, die durch das Hydrogel migriert, in Hohlräumen ansammelt.

Das mindestens eine Hydrogel bzw. die Hydrogelstruktur kann mindestens zwei verschiedene Hydrogele umfassen. Die Hydrogele können insbesondere verschiedene Eigenschaften bezüglich Diffusion von Molekülen, Zellwachstum und Flüssigkeitsdurchlässigkeit aufweisen.

Das bzw. mindestens eines der, insbesondere alle, Hydrogele bzw. die Hydrogelstruktur können Zellen und/oder Zellansammlungen, beispielsweise Zellspheroide, umfassen. Bei den Zellen kann es sich beispielsweise um Tumorzellen und/oder Endothelzellen und/oder Nierenzellen und/oder Leberzellen und/oder Neuronen handeln.

Das mindestens eine Hydrogel bzw. die Hydrogelstruktur kann im polymerisierten Zustand transparent oder transluzent sein. Das mindestens eine Hydrogel bzw. die Hydrogelstruktur kann für Zellen migrierbar oder nicht migrierbar sein. Als migrierbar werden dabei Hydrogele bezeichnet, in denen Zellen, insbesondere Tumorzellen, Fibroblasten, Endothelzellen, in einem Zeitraum von 10 Stunden um einen Zelldurchmesser (beispielsweise einen mittleren Zelldurchmesser für diesen Zelltyp) in eine beliebige Richtung migrieren (auch als Migrationsgeschwindigkeit bezeichnet).

Das mindestens eine Hydrogel bzw. die Hydrogelstruktur kann ein synthetisches Hydrogel umfassen. Das mindestens eine Hydrogel bzw. die Hydrogelstruktur kann ein Hydrogel umfassen, das durch kovalente Vernetzung polymerisiert wird. Beispiele dafür sind Hydrogele, die Polymethylmethacrylat oder, insbesondere funktionalisiertes, Polyethylenglykol (PEG) umfassen. PEG ist kostengünstig und für die Verwendung in Medikamenten zugelassen. PEG wird zudem als sehr biokompatibel eingestuft. Die PEG-Monomere können an ihren Enden mit funktionellen Gruppen wie Acrylsäureestern, Methacrylsäureestern, Vinylcarbonaten, Vinylcarbamaten oder anderen reaktiven En-Funktionalitäten versehen sein. Diese reagieren bei Lichteinwirkung und dem Einsatz eines Photoinitiators über eine Radikalkettenreaktion miteinander und bilden vernetzte Hydrogele aus.

Des Weiteren kann das mindestens eine Hydrogel bzw. die Hydrogelstruktur Copolymere, beispielsweise mit Thiol-Gruppen als reaktive Spezies umfassen. Dabei reagieren z.B. mehrarmige PEG-Monomere mit Norbornen-Endgruppen als reaktive En-Komponente mit den Thiol-Gruppen über einer radikalischen Click-Reaktion zu einem Thioether. So kann eine besonders homogene Maschengröße erhalten werden. Außerdem kann durch die Variation des Molekulargewichts der eingesetzten Monomere, der Menge der verwendeten Monomere und dem Verhältnis unterschiedlicher Monomere zueinander Elastizität, Quellverhalten und Maschenweite des Hydrogels kontrolliert werden. Die Elastizität (G') des Hydrogels, ausgedrückt in Form des Young'schen Moduls, kann beispielsweise zwischen 0,1 bis 100 kPa, insbesondere zwischen 0,05 und 40 kPa, insbesondere zwischen 0,05 und10 kPa, insbesondere zwischen 0,1 und 3 kPa liegen. Das Quellverhalten kann durch das Schwellverhältnis quantisiert werden, also als Verhältnis von der Masse des gequollenen Hydrogels zur Masse des getrockneten Gels, wobei das Quellverhältnis zwischen 5 und 100, insbesondere zwischen 5 und 50, insbesondere zwischen 10 und 40 liegen kann. Die Maschenweite kann zwischen 10 nm und 60 nm liegen. Durch den Einsatz von Monomergemischen können außerdem verschiedene Funktionen in das Hydrogel eingebaut werden. Der Maschenweite wird entsprechend der gängigen Definition verwendet, also als Abstand zwischen zwei benachbarten Knoten, gemessen von Knotenmitte zu Knotenmitte, wobei die Vernetzungsstellen im Hydrogel in diesem Fall den Knoten entsprechen.

Das fotodepolymerisierbare Hydrogel, kann ein spontan gelierendes Gel umfassen. Eine spontane Polymerisierung kann beispielsweise mittels Temperaturverringerung (beispielsweise bei Agarose) oder Temperaturerhöhung (beispielsweise bei Matrigel) oder pH-Änderung (beispielsweise bei Collagen 1) erzielt werden.

Ein Hydrogel kann wellenlängenabhängig zugleich fotopolymerisierbar und fotodepolymerisierbar sein. Die Polymerisierung und die Depolymerisierung erfolgen dann jeweils bei Belichtungen mit Licht unterschiedlicher Wellenlängen.

Für das Polymerisieren der Hydrogele können Acrylate verwendet werden, welche über eine Redox-Aktivierung miteinander reagieren. Auch Click-Reaktionen mit Aziden und Cyclooctin-Derivaten sind möglich. Werden anstelle von Cyclooctinen einfache Alkine verwendet kann eine zusätzliche Aktivierung mit Kupfer-Katalysatoren erfolgen. Auch eine Oxidation von Thiolen kann für die Polymerisierung verwendet werden, indem Disulfidbrücken gebildet werden.

Zur Depolymerisierung von Hydrogelen durch Licht können Nitrobenzoyl-Gruppen verwendet werden, die beispielsweise Licht im Bereich von 325 bis 415 nm absorbieren und dabei gespalten werden. Hydrogele die durch Disulfid-Brücken gebildet werden, können unter Einwirkung eines Photoinitiators ebenfalls fotodepolymerisiert werden. Coumarin-Derivate bilden eine weitere fotodepolymerisierbare Gruppe.

Über eine Thiol-en Click-Reaktion mit einem Photoinitiator und Belichtung bei 450 bis 550 nm kann ein Molekül an das Hydrogel gebunden werden, welches eine Nitrobenzoyl-Gruppe enthält. Dadurch ist sowohl die Anbindung (450 bis 550 nm) als auch das Ablösen (365 nm) des Moleküls durch Belichtung mit der entsprechenden Wellenlänge möglich.

Peptidsequenzen welche eine oder mehrere Cysteine beinhalten oder sonstige Polythiolmoleküle können durch die Click-Reaktion kovalent in das Hydrogel integriert werden. So kann beispielsweise durch den Einbau unterschiedlicher RGD-enthaltender Peptidsequenzen Zelladhäsion an Hydrogele untersucht werden. Auch weitere Funktionen der extrazellulären Matrix, wie die Degradierbarkeit durch beispielsweise Matrix-Metalloproteasen (MMP), kann durch die Verwendung geeigneter Peptidsequenzen nachgebildet werden. In dem Hydrogel können hydrolyseempfindlicher Gruppen wie Polylactat (PLA) oder Thioether beta-ständig zu einer Esterfunktion, welche beispielsweise durch Reaktion von PEG-diacrylat (PEGDA) mit Dithiothreitol (DTT) entstehen, enthalten sein. Diese können zu einem kontinuierlichen Abbau des Hydrogels führen.

Das Substrat kann plattenförmig ausgebildet sein. Plattenförmig bedeutet insbesondere, dass die Höhe des Substrats deutlich kleiner als seine Länge oder seine Breite ist, wobei das Verhältnis von Höhe zu Breite bzw. zu Länge insbesondere kleiner 0,5, vorzugsweise kleiner 0,3, vorzugsweise kleiner 0,15, ist.

Es sei angemerkt, dass in dieser Anmeldung, wenn Verhältnisse angegeben werden, die zuerst genannte Größe den Zähler und die zweite Größer den Nenner darstellt.

Das Substrat kann einteilig oder mehrteilig ausgebildet sein. Das Substrat kann insbesondere mindestens eine Grundplatte und eine Deckplatte umfassen. Im bestimmungsgemäßen Gebrauch ist die Grundplatte unten, also unterhalb der Deckplatte, angeordnet. Die Grundplatte kann, bezogen auf ihre größte Fläche, kleiner als die Deckplatte, bezogen auf die größte Fläche, sein. Die Deckplatte und/oder die Grundplatte können Seitenwände umfassen. Alternativ oder zusätzlich können Seitenwände zusätzlich zur Grundplatte und Deckplatte vorliegen. Die Seitenwände können zur Grundplatte und/oder Deckplatte senkrecht sein.

Die Öffnungen nach außen können in Form von Durchgangslöchern im Substrat ausgebildet sein. Die Öffnungen nach außen können in jeder Seite des Substrats, insbesondere alle in genau einer Seite des Substrats, ausgebildet sein. Die Öffnungen nach außen können derart ausgebildet sein, dass sie im bestimmungsgemäßen Gebrauch nach oben weisen. Die Öffnungen nach außen können in der Deckplatte und/oder der Grundplatte des Substrats vorliegen, insbesondere genau alle in der Deckplatte. Sofern Seitenwände nicht Teil der Deckplatte und der Grundplatte sind, können die Öffnungen nach außen alternativ oder zusätzlich in den Seitenwänden ausgebildet sein.

Die Deckplatte kann einteilig ausgebildet sein und eine erste Seite (eine der beiden größten Flächen) der Deckplatte kann die Decke und die Seitenwände des mindestens einen Fluidkanals oder die Seitenwände des Hohlraums umfassen. Zusätzlich kann die Deckplatte die Öffnungen nach außen in Form von Durchgangslöchern umfassen. Die Deckplatte kann Aufsätze oder Reservoire aufweisen, die die Öffnungen nach außen umgeben. Diese Aufsätze oder Reservoire können insbesondere auf der der ersten Seite gegenüber liegenden zweiten Seite ausgebildet sein. Die Aufsätze oder Reservoire sind über die Öffnungen nach außen mit dem mindestens einen Fluidkanal verbunden. Dies ermöglicht, dass auf geeignete Weise Flüssigkeiten in den mindestens einen Fluidkanal eingebracht werden können. An einer derartigen Deckplatte kann eine Grundplatte, insbesondere in Form einer Kunststofffolie, befestigt sein.

Die Aufsätze oder Reservoire können insbesondere derart ausgebildet sein, dass sie mit Schläuchen verbunden werden können. Die Aufsätze oder Reservoire können insbesondere als Anschlüsse für eine Pumpe, beispielsweise einer Luftpumpe, ausgebildet sein. Die Aufsätze oder Reservoire können beispielsweise innenseitig einem weiblichen Luer-Adapter entsprechen.

Mindestens ein Teil des Substrats, insbesondere die Grundplatte, kann eine Dicke zwischen 1 µm und 2 mm, insbesondere zwischen 50 µm und 300 µm, insbesondere zwischen 100 µm und 250 µm, insbesondere 140 µm bis 170 µm, aufweisen. Beispielsweise kann Borsilikatglas als Grundplatte verwendet werden, z.B. mit einer Stärke zwischen 120 µm und 190 µm, insbesondere mit 140 µm oder 170 µm. Die Deckplatte kann eine Dicke von größer oder gleich 1 mm aufweisen.

Das Substrat bzw. ein oder alle Teile des Substrats können in Form von Spritzgussteilen ausgebildet sein, oder durch Auftragen und/oder Abtragen von Material und/oder Verbinden mehrerer Bestandteile ausgebildet sein. Insbesondere können in einem Kunststoffstück Vertiefungen ausgebildet sein, die mit einer Kunststofffolie abgedeckt werden. So können beispielsweise der Kanal und die Kammer gebildet werden.

Das Substrat kann eine Glasplatte, beispielsweise aus Borsilikatglas, und/oder eine, Kunststoffplatte und/oder eine Kunststofffolie umfassen. Mögliche Kunststoffe sind beispielsweise COC (Cyclo-Olefin Copolymer), COP (Cyclo-Olefin Polymer), PE (Polyethylen), PS (Polystyrol), PC (Polycarbonat) oder PMMA (Polymethylmetacrylat). Insbesondere kann die Grundplatte in Form einer Kunststofffolie ausgebildet sein.

Mindestens ein Teil des Substrats, insbesondere die Grundplatte und/oder die Deckplatte des Substrats, können transparent ausgebildet sein, insbesondere in einem Bereich oberhalb und unterhalb der Kammer bzw. der Hydrogelstruktur. Das Substrat insbesondere die Grundplatte und/oder die Deckplatte des Substrats, kann einen Kunststoff, insbesondere ohne Doppelbrechung und/oder mit einer Eigenfluoreszenz, die im Wesentlichen gleich der Eigenfluoreszenz eines herkömmlichen Deckglases ist, umfassen, insbesondere in einem Bereich oberhalb und unterhalb der Kammer bzw. der Hydrogelstruktur.

Die oben genannten Eigenschaften sind für optische Anwendungen, insbesondere für präzise Fotopolymerisierung und für hochauflösende Mikroskopie besonders vorteilhaft.

Wenn das Substrat mehrteilig ist, können die Bestandteile mittels Klebemittel, Lösungsmittel, UV-Behandlung, radioaktive Behandlung, Laserbehandlung oder thermischem Verschweißen verbunden sein. Das thermische Verschweißen kann flächig oder streifenförmig, insbesondere nur entlang des Rands des Substrats, erfolgt sein. Dies ermöglicht in vorteilhafter Weise eine feste Verbindung.

Das Substrat kann ganz oder teilweise funktionalisiert sein, um das polymerisierte Hydrogel bzw. die Hydrogelstruktur, insbesondere chemisch, an das Substrat zu binden. Eine Funktionalisierung erhöht die Haftung des Hydrogels an dem Substrat.

Die Summe der Oberflächen aller Öffnungen der Kammer bzw. der beiden Oberflächenbereiche zu dem mindestens einen Fluidkanal kann zwischen 0,002 mm² und 200 mm² liegen, insbesondere zwischen 0,05 mm² und 5 mm², insbesondere zwischen 0,2 mm² und 1,6 mm². Insbesondere kann die Oberfläche aller Öffnungen der Kammer bzw. des Oberflächenbereichs zu je einem Teilbereich des genau einen Fluidkanals bzw. die Oberfläche aller Öffnungen der Kammer bzw. des Oberflächenbereichs zu einem der beiden Fluidkanäle jeweils zwischen 0,001 mm² und 100 mm² liegen, insbesondere zwischen 0,1 mm² und 10 mm², insbesondere zwischen 0,4 mm² und 3,2 mm². Die Größe jeder einzelnen Öffnung kann zwischen 0,0005 mm² und 50 mm², insbesondere zwischen 0, 025 mm² und 8 mm², insbesondere zwischen 0,2 mm² und 1,6 mm² liegen.

Die oben genannten Merkmale ermöglichen jeweils, die Menge von Partikeln, die aus einer Flüssigkeit in dem mindestens einen Fluidkanal in das Hydrogel eindringen, strukturell zu beeinflussen. Insbesondere ermöglicht eine große Oberfläche, eine hohe Eintrittsrate von Partikeln in das Hydrogel.

Das Volumen der Kammer bzw. der Hydrogelstruktur kann beispielsweise als Quader, als Würfel, als Prisma, als Zylinder, als Kegel, als Pyramide, als Kegelstumpf oder als Pyramidenstumpf ausgebildet sein. Wenn das Volumen als Prisma ausgebildet ist, kann es sich insbesondere um ein Prisma mit dreieckiger, viereckiger, oder einer anderen vieleckigen Grundfläche handeln. Die Grundfläche ist die Fläche des Bodens der Kammer bzw. der Grenzfläche zwischen der Hydrogelstruktur und dem Substrat.

Insbesondere kann die Kammer eine Decke und einen Boden haben, die jeweils durch die parallel liegende Deckplatte und Grundplatte des Substrats gebildet werden. Ihre Seitenwände können senkrecht bezogen auf den Boden sein oder in einem Winkel von bis zu +/- 20°, insbesondere bis zu +/- 10°, insbesondere bis zu +/- 5° bezogen auf den Boden geneigt sein.

Bei einer Kammer bzw. einer Hydrogelstruktur lassen sich ihre Abmessungen durch ihre Breite, Länge und Höhe beschreiben. Die Höhe der Kammer kann zwischen 0,01 mm und 1 mm liegen, insbesondere zwischen 0,1 mm und 0,5 mm, insbesondere zwischen 0,1 mm und 0,4 mm. Die Länge der Kammer kann zwischen 0,1 mm und 50 mm liegen, insbesondere zwischen 0,5 mm und 10 mm, insbesondere zwischen 1 mm und 4 mm. Die Breite der Kammer kann zwischen 0,05 mm und 20 mm liegen, insbesondere zwischen 0,2 mm und 5 mm, insbesondere zwischen 0,5 mm und 1 mm.

Das Verhältnis von der Höhe zur Breite der Kammer bzw. der Hydrogelstruktur kann zwischen 0,0005 und 20 liegen, insbesondere zwischen 0,02 und 2,5, insbesondere bei 0,2. Das Verhältnis von der Höhe der Kammer bzw. der Hydrogelstruktur zur Länge der Kammer bzw. der Hydrogelstruktur kann zwischen 0,0002 und 10 liegen, insbesondere zwischen 0,01 und 1, insbesondere bei 0,2. Höhe, Länge und Breite können räumlich konstant oder variabel sein. Wenn sie räumlich konstant sind ergibt sich der Spezialfall einer quaderförmigen Kammer bzw. Hydrogelstruktur.

Die Breite und Länge sind Ausdehnungen in einer Ebene parallel zur Grundfläche des Substrats, insbesondere der Grundplatte, wobei die Länge der Ausdehnung in einer Richtung entlang der Fluidkanäle und die Breite der Ausdehnung in einer Richtung senkrecht zu den Fluidkanälen entspricht. Die Höhe wird in einer Ebene senkrecht zur Grundfläche des Substrats gemessen. Die Grundfläche des Substrats ist die Fläche, welche im bestimmungsgemäßen Gebrauch nach unten weist.

Das Volumen der Kammer bzw. der Hydrogelstruktur kann zwischen 0,00005 mm³ und 1000 mm³ liegen, insbesondere zwischen 0,01 mm³ und 25 mm³, insbesondere zwischen 0,1 mm³ und 1,6 mm³.

Die oben beschriebenen Höhenbereiche ermöglichen, dass mehrere Zelllagen übereinander angeordnet sein können, sodass ein dreidimensionales Zellgewebe entsteht, das dennoch eine ausreichend geringe Dicke aufweist, dass die Zellprobe ähnlich wie eine zweidimensionale Zellprobe mikroskopiert werden kann. Man hat also den Vorteil dreidimensional angeordnete Zellen, die sich physiologisch natürlich verhalten können, in einer ausreichend dünnen Schicht beobachten zu können.

Der mindestens eine Fluidkanal kann eine Länge zwischen 0,5 mm und 70 mm haben, insbesondere zwischen 5 mm und 60 mm, insbesondere zwischen 15 mm und 50 mm. Der mindestens eine Fluidkanal kann eine Breite zwischen 0,05 mm und 10 mm, insbesondere zwischen 0,5 mm und 8 mm, insbesondere zwischen 1 mm und 5 mm, haben. Der mindestens eine Fluidkanal kann eine Höhe 0,01 mm und 2 mm, insbesondere zwischen 0,1 und 1 mm, insbesondere zwischen 0,2 mm und 0,5 mm, haben.

Das Verhältnis von der Breite des mindestens einen Fluidkanals jeweils zur Länge des mindestens einen Fluidkanals kann zwischen 0,0007 und 20 liegen, insbesondere zwischen 0,008 und 1,6, insbesondere zwischen 0,02 und 0,3, insbesondere bei 0,2.

Die Definition von Decke, Boden, Länge, Breite und Höhe des mindestens einen Fluidkanals entspricht der Definition von Decke, Boden, Länge, Breite und Höhe der Kammer bzw. Hydrogelstruktur.

Der mindestens eine Fluidkanal kann jeweils einen runden oder ovalen Querschnitt oder einen vieleckigen Querschnitt (in einer Ebene senkrecht zu der Grundfläche des Substrats) haben, insbesondere einen rechteckigen oder trapezförmigen Querschnitt. Der mindestens eine Fluidkanal kann unverzweigt sein oder eine oder mehrere Verzweigungen aufweisen. Die Form der Querschnittsfläche kann entlang des mindestens einen Fluidkanals konstant oder variabel sein. Verschiedene Querschnitte können je nach Herstellungsverfahren und angestrebtem Untersuchungsverfahren von Vorteil sein.

In einer Draufsicht oder Ansicht von unten kann jeder Fluidkanal jeweils einen vieleckigen, insbesondere rechteckigen oder trapezförmigen Unriss haben. Der mindestens eine Fluidkanal kann gerade, gebogen oder abgewinkelt verlaufen. Wenn das Fluidkanalsystem zwei Fluidkanäle umfasst, können diese, insbesondere im Bereich der Kammer bzw. Hydrogelstruktur, parallel verlaufen.

Beispielsweise kann der mindestens eine Fluidkanal von Seitenwänden, einem Boden und einer Decke begrenzt sein. Das Innenvolumen kann beispielsweise quaderförmig, sein. Die Seitenwände können senkrecht bezogen auf den Boden des mindestens einen Fluidkanals sein oder in einem Winkel von bis zu +/- 20°, insbesondere bis zu +/- 10°, insbesondere bis zu +/- 5° bezogen auf den Boden des jeweiligen Fluidkanals geneigt sein. Dies kann bei der Herstellung des Fluidkanals mittels Spritzguss das Abformen vereinfachen. Alternativ kann jeder Fluidkanal jeweils in Form eines runden oder ovalen Rohrs ausgebildet sein.

Der Querschnitt des mindestens einen Fluidkanals kann jeweils eine Fläche zwischen 0,005 mm² und 20 mm² haben, insbesondere zwischen 0,05 mm² und 8 mm², insbesondere zwischen 0,2 mm² und 2,5 mm². So kann die Größe des mindestens einen Fluidkanals beispielsweise an die Viskosität des flüssigen Hydrogels bzw. später verwendeter Lösungen angepasst werden.

Jeder Fluidkanal kann frei von Hydrogel sein.

Die Kammer bzw. die Hydrogelstruktur kann vollständig zwischen einem ersten und einem zweiten Fluidkanal angeordnet sein bzw. in drei Richtungen vollständig von dem einzigen Fluidkanal umgeben sein.

Das Verhältnis von der Länge der Kammer bzw. der Hydrogelstruktur zu der Länge des mindestens einen Fluidkanals kann zwischen 0,001 und 0,75, insbesondere zwischen 0,1 und 0,7 liegen, insbesondere zwischen 0,2 und 0,6, insbesondere bei 0,5. Dies ermöglicht, dass die Kammer bzw. die Hydrogelstruktur auf ihrer gesamten Länge mit Lösung in Kontakt bringbar ist.

Die Höhe des mindestens einen Fluidkanals kann größer oder gleich der Höhe der Kammer bzw. der Hydrogelstruktur sein. Das Verhältnis von der Höhe der Kammer bzw. der Hydrogelstruktur zu der Höhe des mindestens einen Fluidkanals kann zwischen 0,01 und 1,5 liegen, insbesondere zwischen 0,1 und 1, insbesondere zwischen 0,2 und 0,5.

Die obigen Abmessungen ermöglichen, dass die Kammer bzw. die Hydrogelstruktur auf ihrer gesamten Höhe mit Lösung in Kontakt bringbar ist.

In dem Fluidkanalsystem können die zwei Öffnungen nach außen des mindestens einen Fluidkanals entlang des mindestens einen Fluidkanals einander gegenüber liegend angeordnet sein, insbesondere an gegenüberliegenden Enden des mindestens einen Fluidkanals.

Eine derartige Anordnung der Öffnungen nach außen ermöglicht, Flüssigkeiten, beispielsweise nicht polymerisierten Hydrogele oder Lösungen, in den gesamten Fluidkanal einzuleiten und wieder heraus zu spülen.

In dem einzigen Fluidkanal kann im Bereich zwischen den beiden Teilbereichen des Fluidkanals ein fluidischer Widerstand angeordnet sein. Somit kann ein Druckunterschied zwischen den beiden Öffnungen der Kammer bzw. zwischen den beiden Oberflächenbereichen erzeugt werden, wenn eine Flüssigkeit unter Druck in eine der Öffnungen nach außen eingebracht wird.

Der fluidische Widerstand kann in Form eines Strömungswiderstands ausgebildet sein, beispielsweise in Form eines in den Fluidkanal eingebrachten und am Fluidkanal befestigten Körpers oder in Form einer Verengung des Fluidkanals, und/oder in Form eines Ventils, beispielsweise eines Durchgangsventils, eines Eckventils oder eines Schrägsitzventils.

Die im Zusammenhang mit dem Fluidkanalsystem beschriebenen Vorteile treffen ebenso auf das Verfahren zu und werden im Folgenden nicht wiederholt. Im Zusammenhang mit der Vorrichtung beschriebene Merkmale treffen ebenfalls auf das Verfahren zu.

Das erfindungsgemäße Verfahren zum Herstellen eines der oben beschriebenen Fluidkanalsysteme umfasst ein Bereitstellen des Substrats mit der Kammer und dem mindestens einen Fluidkanal, ein Befüllen der Kammer mit fotopolymerisierbarem Hydrogel durch den mindestens einen Fluidkanal, selektives Belichten des Hydrogels, insbesondere unter Verwendung einer Schattenmaske, sodass in der Kammer befindliches Hydrogel zumindest teilweise fotopolymerisiert wird, und ein Spülen jedes Fluidkanals zum Entfernen von nicht polymerisiertem Hydrogel.

Ein weiteres erfindungsgemäßes Verfahren zum Herstellen eines der oben beschriebenen Fluidkanalsysteme umfasst ein Bereitstellen des Substrats mit der Kammer und dem mindestens einen Fluidkanal, ein Befüllen der Kammer mit fotodepolymerisierbarem Hydrogel durch den mindestens einen Fluidkanal, Polymerisieren des Hydrogels, selektives Belichten des Hydrogels, insbesondere unter Verwendung einer Schattenmaske, sodass in jedem Fluidkanal befindliches Hydrogel fotodepolymerisiert wird, und ein Spülen jedes Fluidkanals zum Entfernen von nicht polymerisiertem Hydrogel.

Es sei hierbei angemerkt, dass diese beiden Verfahren sich jeweils auf die Varianten des Fluidkanalsystems mit einer Kammer und mindestens einem Fluidkanal beziehen.

Ein weiteres erfindungsgemäßes Verfahren zum Herstellen eines der oben beschriebenen Fluidkanalsysteme umfasst ein Bereitstellen des Substrats mit dem Hohlraum, ein Befüllen des Hohlraums mit fotopolymerisierbarem Hydrogel, ein selektives Belichten des Hydrogels, insbesondere unter Verwendung einer Schattenmaske, sodass in dem Hohlraum befindliches Hydrogel zumindest teilweise fotopolymerisiert wird, derart, dass der mindestens eine Fluidkanal und die zusammenhängende Hydrogelstruktur ausgebildet werden, und ein Spülen jedes Fluidkanals zum Entfernen von nicht polymerisiertem Hydrogel.

Ein weiteres erfindungsgemäßes Verfahren zum Herstellen eines der oben beschriebenen Fluidkanalsysteme umfasst ein Bereitstellen des Substrats mit dem Hohlraum, ein Befüllen des Hohlraums mit fotodepolymerisierbarem Hydrogel, Polymerisieren des Hydrogels, selektives Belichten des Hydrogels, insbesondere unter Verwendung einer Schattenmaske, sodass in dem Hohlraum befindliches Hydrogel zumindest teilweise fotodepolymerisiert wird, derart, dass der mindestens eine Fluidkanal und die zusammenhängende Hydrogelstruktur ausgebildet werden, und ein Spülen jedes Fluidkanals zum Entfernen von nicht polymerisiertem Hydrogel.

Es sei hierbei angemerkt, dass diese beiden Verfahren sich jeweils auf die Varianten des Fluidkanalsystems mit einem Hohlraum beziehen, in dem Hydrogel angeordnet ist, das derart strukturiert ist, dass mindestens ein Fluidkanal und eine zusammenhängende Hydrogelstruktur ausgebildet sind. Das selektive Belichten bezieht sich auf ein räumlich selektives Belichten.

Die Fotopolymerisierung bzw. Fotodepolymerisierung hat den Vorteil, dass das Hydrogel zu einem wählbaren Zeitpunkt in kurzer Zeit und räumlich definiert polymerisiert werden kann.

Das Ausbilden des mindestens einen Fluidkanals und der Hydrogelstruktur kann in beliebiger Reihenfolge erfolgen.

Das Spülen kann mittels einer Spülflüssigkeit, beispielsweise destillierten Wassers oder eines Spülpuffers, beispielsweise phosphatgepufferte Salzlösung (PBS), oder durch Ersetzen des nicht-polymerisierten Hydrogels durch eine Flüssigkeit, beispielsweise Puffer, insbesondere PBS, oder Zellmedium bzw. Nährmedium, insbesondere Dulbecco's Modified Eagle's Medium (DMEM), erfolgen. Die (Spül-)Flüssigkeit kann durch eine der beiden Öffnungen nach außen in jeden Fluidkanal eingeleitet werden, insbesondere unter Druck, und das nicht-polymerisierte Hydrogel kann durch die zweite Öffnung nach außen aus jedem Fluidkanal gespült werden.

Die Schattenmaske kann in Form einer ebenen Platte aus einem, zumindest für UV-Strahlung, transparenten Material ausgebildet sein. Die Schattenmaske kann als Binärmaske ausgebildet sein, die in einigen Bereichen mit einer nicht-transparenten Schicht beschichtet ist. Die Schattenmaske kann eine aus dem Bereich der Fotolithografie bekannte Schattenmaske sein. Beispielsweise kann es sich um eine Chrommaske, also einer Maske mit einer Chromschicht auf einer Glasplatte (chrom on glas, COG) oder ein Maske mit einer opaken MoSi-Schicht auf einer Glasplatte (opaque MoSi on glass, OMOG) handeln. Alternativ kann auch eine Phasenmaske verwendet werden.

Das Belichten kann mittels einer Lichtquelle erfolgen, beispielsweise einer UV-Lampe. Insbesondere kann eine Lichtquelle mit einer Wellenlänge im Bereich von 315 nm bis 600 nm, insbesondere 340 nm bis 400 nm, insbesondere 365 nm, verwendet werden. Die Leistung kann zwischen 1 mW/cm² und 100 mW/cm², insbesondere zwischen 1 mW/cm² und 20 mW/cm² insbesondere zwischen 4 mW/cm² und 10 mW/cm² betragen. Typische Belichtungsdauern liegen bei 1 s bis 420 s, insbesondere 1 s bis 60 s, insbesondere 5 s bis 30 s, wobei die Belichtungsdauer umso niedriger ist, je höher die Leistung ist.

Das Licht kann senkrecht oder schräg auf das Hydrogel einfallen. Bei senkrechtem Lichteinfall lassen sich beispielsweise Hydrogelstrukturen wie Quader oder Würfel erzeugen. Bei schrägem Lichteinfall können Hydrogelstrukturen mit schrägen Seitenwänden hergestellt werden. Es kann ein optischer Aufbau zum Parallelisieren des Lichts oder ein optischer Aufbau zum Bündeln des Lichts vorgesehen sein. Paralleles Licht ermöglicht eine genaue Abbildung der Struktur der Schattenmaske auf das Hydrogel. Das Licht kann auch gebündelt werden. Beispielsweise kann von unten parallel einfallendes Licht mittels einer Sammellinse auf einen Brennpunkt, der auf der Oberfläche des nicht-polymerisierten Gels liegt, gebündelt werden. Bei der Verwendung kreisförmiger lichtundurchlässiger Bereiche auf der Maske entstehen dann beispielsweise Kegel oder Kegelstümpfe aus polymerisiertem Hydrogel.

Das Belichten kann senkrecht oder schräg von unten erfolgen. Über der Lichtquelle, und gegebenenfalls über dem optischen Aufbau, ist die Schattenmaske und darüber das mit unpolymerisiertem Hydrogel gefüllte Substrat angeordnet. Die Belichtung kann alternativ senkrecht oder schräg von oben oder von der Seite erfolgen. Das Licht kann durch weitere optische Elemente, beispielsweise Umlenkspiegel, geleitet werden. Dies kann ermöglichen, die optische Anordnung kompakter oder in anderer Weise vorteilhaft zu gestalten. Es ist vorteilhaft, die Maske bei der Belichtung möglichst nah an das Substrat anzunähern, insbesondere in direkten Kontakt mit dem Substrat zu bringen, gegebenenfalls sogar unter Druckbeaufschlagung. So wird eine gute optische Abbildung der Strukturen der Maske auf das Hydrogel erzielt.

Das Belichten kann derart erfolgen, dass das Hydrogel in der Kammer bzw. die Hydrogelstruktur strukturiert ist, insbesondere so strukturiert, dass in dem Hydrogel Kanäle ausgebildet werden, insbesondere Kanäle, die den ersten Fluidkanal und den zweiten Fluidkanal bzw. die beiden Teilbereiche des einzigen Fluidkanals miteinander verbinden. Alternativ oder zusätzlich können in dem Hydrogel Hohlräume ausgebildet werden, die insbesondere miteinander verbunden sein können.

Die Maske kann derart ausgebildet sein, dass das Hydrogel in jedem Fluidkanal nicht belichtet wird. Dabei kann ein fotopolymerisierbares Hydrogel verwendet werden. Somit ist jeder Fluidkanal nach dem Spülen frei von Hydrogel. Die Maske kann alternativ derart ausgebildet sein, dass das Hydrogel in jedem Fluidkanal belichtet wird. Dabei kann ein fotodepolymerisierbares Hydrogel verwendet werden. Somit ist jeder Fluidkanal nach dem Spülen frei von Hydrogel.

Die Höhe des Hydrogels kann durch die Menge des eingeleiteten nicht-polymerisierten Hydrogels, also mittels des Füllstands, gesteuert werden.

Das Ausbilden des mindestens einen Fluidkanals kann ein Ausbilden genau eines, mindestens zweier, insbesondere genau zweier, Fluidkanäle umfassen.

Das Verfahren kann, vor dem Befüllen, eine vollständige oder teilweise Funktionalisierung der Oberfläche des Substrats umfassen, um das Hydrogel, insbesondere chemisch, beispielsweise kovalent, an die Oberfläche des Substrats, insbesondere an die Wände der Kammer, zu binden. Bei der Bindung kann es sich insbesondere um eine spezifische Bindung handeln. Die Bindung kann die Ablösung von Hydrogelen von dem Substrat verhindern.

Insbesondere können Bindungsmoleküle, beispielsweise heterobifunktionale PEG-Linker oder funktionalisierte Silane, auf die Oberfläche des Substrats aufgebracht werden, die an der Oberfläche des Substrats kovalent binden. Die Bindungsmoleküle können an der Oberfläche die gleichen Bindungsgruppen aufweisen, die im Hydrogel für die Fotopolymerisierung verwendet werden. In diesem Fall entsteht bei der Fotopolymerisierung gleichzeitig eine kovalente Bindung zur Oberfläche. Das Anbinden des Hydrogels an die Oberfläche kann also im gleichen Schritt erfolgen wie die Fotopolymerisierung. Es ist aber auch möglich, die Fotopolymerisierung und die Anbindung an die Oberfläche unabhängig voneinander durchzuführen, beispielsweise indem eine von der Vernetzung des Hydrogels unabhängige Chemie zur Bindung an die Oberfläche verwendet wird. Das Anbinden des Hydrogels an die Oberfläche kann also in einem gesonderten Schritt erfolgen.

Das fotopolymerisierbare bzw. das fotodepolymerisierbare Hydrogel kann ein erstes fotopolymerisierbares Hydrogel bzw. ein erstes fotodepolymerisierbares Hydrogel sein, wobei die Kammer nach dem Befüllen, Belichten und Spülen des ersten polymerisierbaren Hydrogels bzw. des ersten fotodepolymerisierbaren Hydrogels nicht vollständig mit polymerisiertem Hydrogel gefüllt ist. Das Verfahren kann anschließend ein Befüllen der Kammer mit einem zweiten Hydrogel durch den mindestens einen Fluidkanal umfassen, wobei das zweite Hydrogel fotopolymerisierbar oder fotodepolymerisierbar ist. Das Verfahren kann anschließend ein selektives Belichten des zweiten Hydrogels, insbesondere unter Verwendung einer Schattenmaske, umfassen, sodass in der Kammer befindliches nicht-polymerisiertes Hydrogel zumindest teilweise fotopolymerisiert bzw. in jedem Fluidkanal befindliches zweites Hydrogel, insbesondere vollständig, fotodepolymerisiert wird. Das Verfahren kann anschließend ein Spülen jedes Fluidkanals zum Entfernen von nicht polymerisiertem zweitem Hydrogel umfassen.

Das Verfahren kann das Wiederholen der zuvor beschriebenen Verfahren mit verschiedenen Hydrogelen umfassen, bis die Kammer zu mindestens 90%, insbesondere mindestens 95%, insbesondere vollständig, mit polymerisierten Hydrogel gefüllt ist.

Bei dem Verfahren kann das bzw. mindestens eines der fotopolymerisierbaren Hydrogele bzw. der fotodepolymerisierbaren Hydrogele beim Befüllen der Kammer Zellen umfassen. Alternativ können Zellen erst nach dem Fotopolymerisieren bzw. Fotodepolymerisieren des Hydrogels eingebracht werden, beispielsweise durch das Herstellen eines Kontakts zwischen dem Hydrogel und einer Flüssigkeit, die Zellen enthält. Dazu kann die Flüssigkeit, die Zellen enthält, in den mindestens einen Fluidkanal eingebracht werden.

Die Erfindung stellt auch eine Verwendung eines der oben beschriebenen Fluidkanalsysteme bereit.

Die Verwendung eines der oben beschriebenen Fluidkanalsysteme umfasst das Befüllen des bzw. eines ersten der beiden Fluidkanäle durch die Öffnung nach außen mit einer Flüssigkeit, die Partikel enthält, die derart ausgebildet sind, dass sie in das Hydrogel eindringen können, insbesondere Partikel, die mit im Hydrogel enthaltenen Partikeln oder Zellen wechselwirken.

Bei der Flüssigkeit kann es sich beispielsweise um ein Zellmedium, insbesondere DMEM, handeln. Die Flüssigkeit kann beispielsweise Nährstoffe, insbesondere Glucose, Glutamat oder Pyruvat, und/oder im Medium gelösten Gase, beispielsweise Sauerstoff, enthalten, insbesondere Nährstoffe oder Gase, die nötig sind, um Zellen am Leben zu erhalten und/oder um Zellwachstum zu fördern. Die Versorgung der Zellen kann insbesondere statisch, d.h. ausschließlich über Diffusion, oder durch kontinuierlichen Fluss der Flüssigkeit durch den, einen der bzw. die Fluidkanäle erfolgen.

Die Verwendung des Fluidkanalsystems kann das Befüllen des zweiten der beiden Fluidkanäle mit einer zweiten Flüssigkeit durch die Öffnung nach außen umfassen. Die zweite Flüssigkeit kann ebenfalls die in der ersten Flüssigkeit enthaltenen Partikel, insbesondere in einer niedrigeren Konzentration als in der ersten Flüssigkeit, und/oder andere Partikel oder keine Partikel umfassen.

Die Verwendung des Fluidkanalsystems kann weiterhin das Anlegen eines Drucks an den einen bzw. einen ersten und/oder einen zweiten der beiden Fluidkanäle umfassen. Der Druck kann beispielsweise zwischen 0.1 mbar und 300 mbar liegen, insbesondere 5 mbar und 70 mbar, insbesondere zwischen 5 mbar und 60 mbar. Das Anlegen eines Drucks erzeugt einen sogenannten interstitiellen Fluss durch das Hydrogel, der in ähnlicher Weise auch in biologischem Gewebe auftritt.

Je nach Stärke des Metabolismus und Anzahl bzw. Dichte der Zellen in dem Hydrogel reicht eine Versorgung der Zellen lediglich durch Diffusion der Nährstoffe und im Medium gelösten Gase nicht aus. Durch den interstitiellen Fluss lassen sich auch größere Zellansammlungen oder sogar Gewebeabschnitte aus Organismen über längere Zeiträume am Leben erhalten.

Die Verwendung des Fluidkanalsystems kann zusätzlich das Anlegen eines Drucks an den zweiten der beiden Fluidkanäle umfassen, wobei der Druck ungleich dem Druck im ersten der beiden Fluidkanäle ist. Insbesondere kann dabei der Druck in dem Fluidkanal mit einer höheren Partikelkonzentration höher sein, als der Druck in dem Fluidkanal mit einer niedrigeren Partikelkonzentration der gleichen Partikel.

Der erste Fluidkanal kann mit einer ersten Flüssigkeit gefüllt sein und der zweite Fluidkanal kann mit einer zweiten Flüssigkeit gefüllt sein. In der ersten Flüssigkeit können Partikel enthalten sein, die nicht in der zweiten Flüssigkeit enthalten sind und in der zweiten Flüssigkeit können Partikel enthalten sein, die nicht in der ersten Flüssigkeit enthalten sind, insbesondere Partikel, die eine andere Wirkung auf Zellen haben als die Partikel in der ersten Flüssigkeit. Je nachdem in welchem Fluidkanal ein höherer Druck angelegt wird, kann eingestellt werden, welche Partikel bzw. in welchem Verhältnis die verschiedenen Partikel in das Hydrogel eindringen.

Die Verwendung des Fluidkanalsystems kann das Anlegen des Drucks an den bzw. die Fluidkanäle jeweils unter Verwendung einer Pumpe, insbesondere einer Luftpumpe umfassen, die an eine der Öffnungen nach außen des jeweiligen Fluidkanals angeschlossen ist. Die Verwendung des Fluidkanalsystems kann weiterhin umfassen, dass beim Anlegen des Drucks jeweils eine der mindestens zwei Öffnungen nach außen des jeweiligen Fluidkanals verschlossen wird.

Die Verwendung des Fluidkanalsystems kann umfassen, dass Zellen in dem Hydrogel mit Partikeln in der Flüssigkeit versorgt werden, und dass die Zellen, insbesondere deren Wachstum und/oder Migration, untersucht werden, insbesondere mittels optischer Mikroskopie oder Fluoreszenzmikroskopie. Beispielsweise kann beobachtet werden, wie weit Zellen pro Zeiteinheit in ein zellfreies Hydrogel wandern. Aus diesen Daten lassen sich Parameter wie z.B. die Migrationsgeschwindigkeit und die Invasivität der Zellen bestimmen. Nach Zugabe von Partikeln kann man ihren Einfluss auf die entsprechenden Parameter messen.

Eine weitere Verwendungsmöglichkeit des Fluidkanalsystems besteht darin, Endothelzellen oder Epithelzellen als Suspension in einem Puffer in den mindestens einen Fluidkanal einzufüllen und diese zu beobachten. Die Zellen besiedeln die Hydrogelwände und bilden dadurch eine biologisch relevante Begrenzung zu dem Hydrogelbereich. Dadurch simuliert der Fluidkanal Blutgefäße, mit ähnlichen Barriereeigenschaften wie die biologischen Blutgefäßwände.

Das Fluidkanalsystem kann zur Untersuchung von Tumorzellen und zum Erstellen von Modellen für Zellwachstum, Proliferation und/oder Zellmigration genutzt werden. Außerdem können Wirksamkeitsstudien für die Behandlung von Tumoren durchgeführt werden.

Weitere Merkmale und Vorteile werden nachfolgend anhand der beispielhaften Figuren erläutert. Dabei zeigt:
- Figur 1: eine schematische, nicht maßstabsgetreue Ansicht von unten auf die Deckplatte eines Substrats einer ersten Ausführungsform;
- Figur 2: eine schematische, nicht maßstabsgetreue Draufsicht auf die Deckplatte eines Substrats der ersten Ausführungsform;
- Figur 3: eine schematische, nicht maßstabsgetreue Darstellung eines Schnitts durch das Fluidkanalsystem der ersten Ausführungsform;
- Figur 4: eine schematische, nicht maßstabsgetreue Schrägansicht auf das Fluidkanalsystem der ersten Ausführungsform;
- Figur 5: eine schematische, nicht maßstabsgetreue Ansicht von unten auf die Deckplatte eines Substrats einer zweiten Ausführungsform;
- Figur 6: eine schematische, nicht maßstabsgetreue Ansicht von unten auf die Deckplatte eines Substrats einer dritten Ausführungsform.
- Figuren 7 bis 11: eine schematische, nicht maßstabsgetreue Darstellung eines Herstellungsverfahrens für ein Fluidkanalsystem nach einem ersten Ausführungsbeispiel;
- Figuren 12 bis 14: schematische, nicht maßstabsgetreue Darstellungen weiterer Varianten des Herstellungsverfahrens für ein Fluidkanalsystem nach dem ersten Ausführungsbeispiel;
- Figuren 15 bis 17: eine schematische, nicht maßstabsgetreue Darstellung eines Herstellungsverfahrens für ein Fluidkanalsystem nach einem zweiten Ausführungsbeispiel;
- Figur 18: eine schematische, nicht maßstabsgetreue Darstellung einer Schattenmaske zum Herstellen abgewinkelter Fluidkanäle;
- Figur 19: eine schematische, nicht maßstabsgetreue Darstellung einer Schattenmaske, die invers zu der Schattenmaske in Figur 8 ist;
- Figur 20: eine schematische, nicht maßstabsgetreue Darstellung eines ersten Ausführungsbeispiels für die Verwendung der Vorrichtung; und
- Figur 21: eine schematische, nicht maßstabsgetreue Darstellung eines zweiten Ausführungsbeispiels für die Verwendung der Vorrichtung.

Im Folgenden und in den Figuren werden in den verschiedenen Ausführungsbeispielen, sofern nicht anders spezifiziert bis auf das Hochzählen der Hunderterstelle die gleichen Bezugszeichen für gleiche oder entsprechende Elemente verwendet.

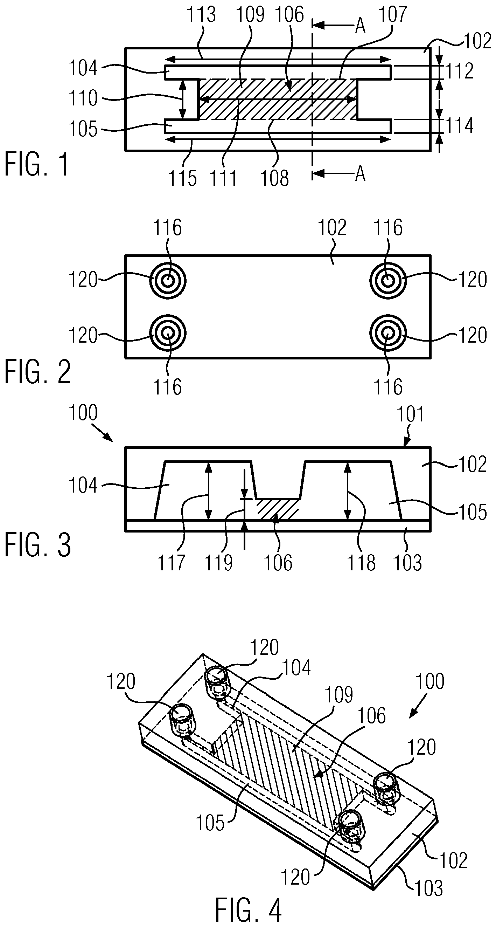

Figuren 1 bis 4 zeigen verschiedene Elemente des erfindungsgemäßen Fluidkanalsystems 100 nach einer ersten Ausführungsform. Insbesondere zeigt Figur 1 einen Teil eines plattenförmigen, transparenten Kunststoffsubstrats 101, in diesem Fall die Deckplatte 102 des Kunststoffsubstrats, in einer Ansicht von unten und Figur 2 zeigt die Deckplatte 102 in Draufsicht. Figur 3 zeigt einen Querschnitt des Fluidkanalsystems mit der Deckplatte 102 und der daran befestigten Grundplatte 103 des Substrats. Die Deckplatte des Kunststoffsubstrats ist in Form eines Kunststoffquaders ausgebildet, in dem wiederum Vertiefungen ausgebildet sind, die mit der im wesentlichen ebenen Grundplatte abgedeckt werden, sodass im Substrat ein erster Fluidkanal 104, ein zweiter Fluidkanal 105 und eine Kammer 106 ausgebildet sind. Wie in der Ansicht von unten in Figur 1 zu erkennen ist, hat die Kammer genau eine Öffnung 107 zum ersten Fluidkanal und genau eine Öffnung 108 zum zweiten Fluidkanal. Sie ist zu dem ersten Fluidkanal und dem zweiten Fluidkanal hin jeweils entlang ihrer gesamten Länge offen. Die Kammer kann jedoch auch jeweils mehrere Öffnungen zu dem ersten und/oder dem zweiten Fluidkanal haben, beispielsweise kann sie zu den Fluidkanälen hin unterbrochene Wände aufweisen.

Die Kammer hat in der Draufsicht einen rechteckigen Umriss. Sie kann jedoch beispielsweise auch einen quadratischen, anderweitig vieleckigen, runden oder ovalen Umriss haben. Die Länge 111 der Kammer ist in diesem Beispiel größer als die Breite 110 der Kammer, das Verhältnis von der Breite zur Länge der Kammer ist also kleiner als 1.

In der Kammer ist ein fotopolymerisiertes Hydrogel 109 angeordnet, welches sich über die gesamte Breite und Länge der Kammer erstreckt. Dies ist jedoch nicht zwangsläufig der Fall. Beispielsweise kann das Hydrogel sich nicht zu allen Wänden hin erstrecken. Es kann also beispielsweise eine geringere Länge als die Länge der Kammer haben. Die Kammer muss dennoch zu mindestens 90 % mit Hydrogel gefüllt sein. In der Kammer können auch mehrere verschiedene fotopolymerisierte Hydrogele angeordnet sein, die zusammen die Kammer zu mindestens 90% ausfüllen.

Es sei angemerkt, dass, beispielsweise aufgrund des Herstellungsverfahrens, die Höhe des Hydrogels in der Kammer, beispielsweise durch Bildung eines Meniskus an den Wänden der Kammer, im Bereich der Wände der Kammer größer sein kann als in von den Wänden beabstandeten Bereichen.

Das Hydrogel verhindert in diesem Beispiel eine freie Strömung zwischen den beiden Fluidkanälen bzw. von einem Teilbereich eines einzigen Fluidkanals zu dem anderen Teilbereich des einzigen Fluidkanals durch die Kammer. Alternativ kann das Hydrogel so ausgebildet sein, dass eine Flüssigkeit, die in einen der beiden Fluidkanäle eingefüllt wird bzw. in einen Teilbereich des Fluidkanals eingefüllt wird, durch die Kammer in den anderen Fluidkanal bzw. in den anderen Teilbereich des Fluidkanals strömen kann.

Die Kammer (bzw. die später beschriebene Hydrogelstruktur) kann jeweils auch als Beobachtungsbereich bezeichnet werden, da diese typischerweise Zellen enthält, die bei der Verwendung des Fluidkanalsystems beobachtet werden.

Die Auswahl der Höhe, Breite, Länge und/oder eines bestimmten Volumens der Kammer (bzw. der später beschriebenen Hydrogelstruktur) ermöglichen zudem das Nachstellen bestimmter Randbedingungen beim Zellwachstum. Je nach Größe der Kammer können beispielsweise Modelle für kleine, gut mit Nährstoffen versorgte Tumore (Durchmesser der Mikrotumore maximal 1 mm) entwickelt werden, oder auch Modelle für größere, d.h. mit Nährstoffen teilweise unterversorgte Tumore (Tumorgröße bis maximal 5 mm), die im Inneren nicht nekrotisch sind oder im Inneren nekrotisches Gewebe entwickeln. Die zu wählenden Werte hängen vom Nährstoffverbrauch der beobachteten Zellen, also auch von deren Metabolismus ab.

Wie in der Figur gezeigt ist das Verhältnis von der Breite 112 des ersten Fluidkanals zur Länge 113 des ersten Fluidkanals kleiner als 0,2. Das Verhältnis kann aber auch davon abweichen, der Fluidkanal ist jedoch vorzugsweise länger als breit. Dasselbe gilt für die Breite 114 und die Länge 115 des zweiten Fluidkanals.

Der erste Fluidkanal und der zweite Fluidkanal haben in dieser Ausführungsform in Draufsicht jeweils einen rechteckigen Umriss. Auch hier sind jedoch andere Formen möglich, beispielsweise eine abgewinkelte oder gekrümmte Form.

In Figur 2 ist die Grundplatte 102 des Kunststoffsubstrats in Draufsicht gezeigt. In der Grundplatte sind vier Durchgangslöcher ausgebildet, von denen jeweils zwei die Öffnungen nach außen 116 des ersten bzw. des zweiten Fluidkanals bilden. Außerdem sind Reservoire 120 gezeigt, die als Teil des Substrats an dessen Außenseite die Öffnungen umgebend angeordnet sind und an denen beispielsweise jeweils ein Schlauch oder eine Pumpe befestigt werden kann.

Figur 3 zeigt einen Querschnitt entlang der in Figur 1 eingetragenen Linie A-A durch das erfindungsgemäße Fluidkanalsystem der ersten Ausführungsform. Hier ist zu erkennen, dass der Querschnitt sowohl des ersten als auch des zweiten Fluidkanals und der Kammer jeweils trapezförmig ist. Die Volumen der Kammer bzw. des Fluidkanals bilden jeweils Pyramidenstümpfe. Andere Querschnitte sind jedoch ebenfalls möglich, beispielsweise ein rechteckiger Querschnitt.

Wie hier gezeigt ist hat die Grundplatte 103, die hier beispielsweise in Form einer Folie ausgebildet ist, eine Länge und Breite, die der Länge und Breite der Deckplatte 102 des Kunststoffsubstrats entspricht. Die Folie hat dabei eine Dicke von etwa 0,2 mm.

Die Höhe 117 des ersten Fluidkanals und die Höhe 118 des zweiten Fluidkanals ist jeweils größer als die Höhe 119 der Kammer. Die Kammer kann jedoch auch die gleiche Höhe haben wie die Fluidkanäle. Ebenfalls zu erkennen ist hier, dass das Hydrogel 109 die gesamte Höhe der Kammer einnimmt. Es ist jedoch auch möglich, dass dies nicht der Fall ist, wobei die Kammer dennoch zu mindestens 90 % mit Hydrogel gefüllt sein muss.

Figur 4 zeigt eine Schrägansicht der oben beschriebenen Ausführungsform.

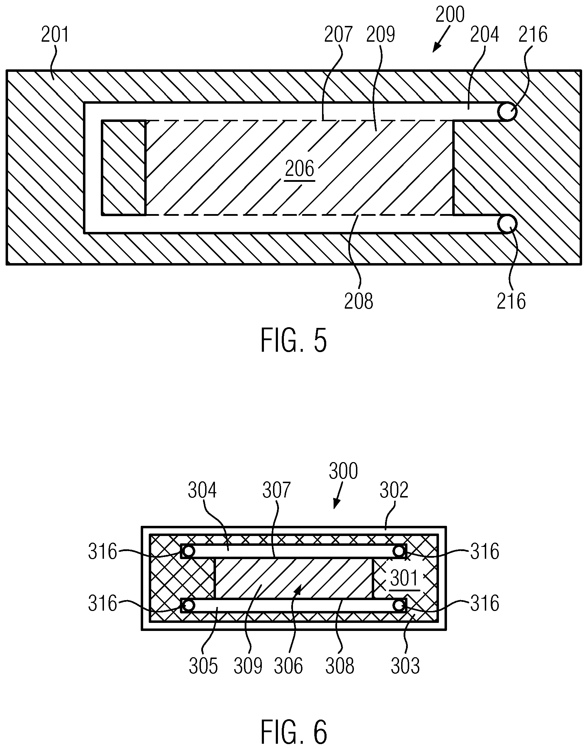

In Figur 5 ist die Ansicht von unten auf die Deckplatte eines Substrats 201 einer zweiten Ausführungsform 200 dargestellt. In diesem Fall umfasst das Fluidkanalsystem genau einen Fluidkanal 204, zu dem die Kammer 206 ausschließlich an zwei gegenüberliegenden Teilbereichen des Fluidkanals über die Öffnungen 207 und 208 der Kammer offen ist. Abgesehen von der Ausführung mit nur einem statt zwei Fluidkanälen, ist das Fluidkanalsystem wie in der ersten Ausführungsform ausgebildet. Daher sind hier kein Querschnitt, keine Draufsicht und keine Schrägansicht mehr gezeigt. Die Form und Abmessungen des Substrats, der Fluidkanäle und der Kammer können jedoch auch von denen in der ersten Ausführungsform abweichen. Ein hier nicht gezeigter fluidischer Widerstand kann zwischen den beiden Schenkeln des Fluidkanals angeordnet sein.

In Figur 6 ist die Ansicht von unten auf die Deckplatte 302 eines Substrats 301 einer dritten Ausführungsform dargestellt, in der das Fluidkanalsystem 300 einen Hohlraum umfasst, der durch die Deckplatte mit Seitenwänden sowie eine Grundplatte (hier nicht gezeigt) aus Glas oder Kunststoff begrenzt wird. Das Substrat ist also zweiteilig ausgebildet. In dem Hohlraum ist ein fotopolymerisiertes Hydrogel 303 angeordnet, in dem ein erster Fluidkanal 304 und zweiter Fluidkanal 305 ausgebildet sind. Dabei ist zwischen dem ersten Fluidkanal und dem zweiten Fluidkanal eine zusammenhängende Hydrogelstruktur 306 angeordnet. Die Hydrogelstruktur grenzt jeweils mit einem Oberflächenbereich an den ersten und den zweiten Fluidkanal an und hat ausschließlich über die beiden Fluidkanäle eine Verbindung nach außen. In der Deckplatte sind vier Durchgangslöcher 316 ausgebildet, die die zwei Öffnungen nach außen des ersten Fluidkanals und die zwei Öffnungen nach außen des zweiten Fluidkanals bilden.

Die Fluidkanäle sind entsprechend der ersten oder zweiten Ausführungsform ausgebildet. Die Hydrogelstruktur ist entsprechend den Abmessungen der Kammer aus der ersten und zweiten Ausführungsform ausgebildet. Die Form und Abmessungen können jedoch auch davon abweichen.

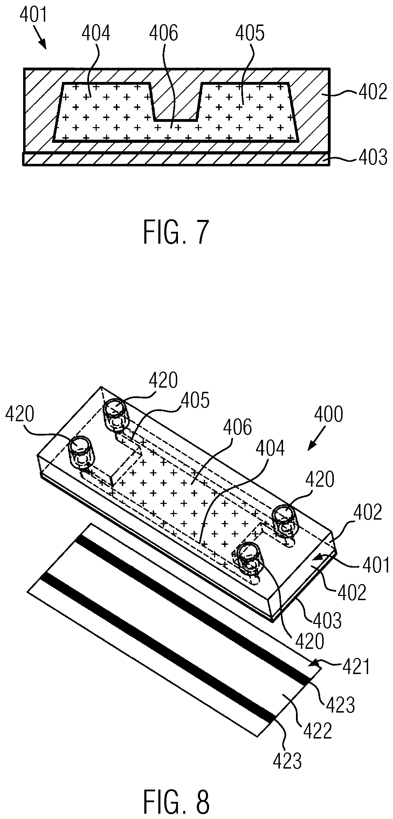

Figuren 7 bis 11 zeigen die Schritte eines erfindungsgemäßen Herstellungsverfahrens für ein Fluidkanalsystem.

In Figur 7 ist ein zweiteiliges Substrat 401 im Querschnitt gezeigt, beispielsweise ein Plastik oder Glassubstrat, in dem zwei Fluidkanäle 404 und 405 und eine Kammer 406 ausgebildet sind, die beispielsweise die Formen und Abmessungen haben können, die in der ersten Ausführungsform der Vorrichtung beschrieben wurden. Durch die hier nicht gezeigten Öffnungen nach außen der Fluidkanäle wird ein flüssiges, nicht-polymerisiertes Hydrogel in die Fluidkanäle und die Kammer eingefüllt, bis die Fluidkanäle und die Kammer vollständig gefüllt sind. Wenn sich das polymerisierte Hydrogel nicht bis zur Decke der Kammer hin erstrecken soll, muss kein vollständiges Befüllen erfolgen.

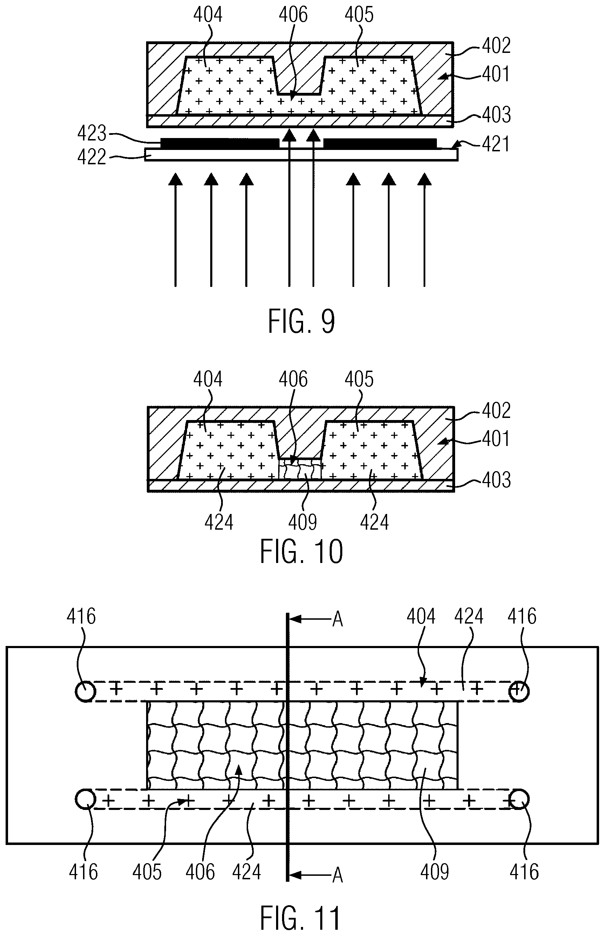

Wie in Figuren 8 und 9 gezeigt, wird in einem nächsten Schritt das Hydrogel mittels Fotopolymerisierung strukturiert. Figur 8 zeigt in einer Schrägansicht, wie eine Schattenmaske 421 von unten an das Substrat angenähert wird. Die Schattenmaske ist in diesem Fall eine Chrommaske, die aus einer transparenten Glasplatte 422 besteht, auf der zwei Chromstreifen 423 in Form einer Chromschicht auf der Glasplatte aufgebracht sind. Die Chromstreifen lassen kein Licht durch. Es muss jedoch keine Chrommaske verwendet werden, sondern jede andere gängige Art von Schattenmaske kann verwendet werden. Die Länge und Breite der Schattenmaske entspricht hier etwa der Länge und Breite des Substrats, kann jedoch auch eine andere Größe haben, beispielsweise wenn das durch den optischen Aufbau erforderlich ist. Die Chromstreifen sind derart geformt und die Schattenmaske wird zur Belichtung derart auf dem Substrat positioniert, dass die Chromstreifen beide Fluidkanäle vollständig abdecken, jedoch die Kammer nicht abdecken.

In Figur 9 ist in einem Querschnitt entlang der in Figur 11 eingetragenen Linie A-A dargestellt, wie paralleles Licht von unten durch die Schattenmaske 421 auf das hydrogelgefüllte Substrat 401 trifft. Wie zu erkennen ist, gelangt nur Licht durch die Maske auf das Substrat, wo keine Chromstreifen 423 im Lichtweg angeordnet sind (wenn man von Beugungseffekten absieht). Dadurch wird in diesem Fall nur das Hydrogel in der Kammer fotopolymerisiert. In der Figur ist zwar ein Abstand zwischen der Maske und dem Substrat angedeutet. In der Praxis ist jedoch ein möglichst geringer Abstand, insbesondere direkter Kontakt vorteilhaft, da dann die Strukturen auf der Schattenmaske präziser auf das Hydrogel abgebildet werden können.

Figuren 10 und 11 zeigen jeweils in einem Querschnitt entlang der Linie A-A bzw. in Draufsicht, dass das Hydrogel 409 in der Kammer polymerisiert ist, während das Hydrogel 424 in den Fluidkanälen nicht polymerisiert ist. In einem hier nicht gezeigten Schritt wird jeweils durch eine Öffnung 416 jedes der beiden Fluidkanäle ein Spülpuffer in die Fluidkanäle eingebracht, und durch die Fluidkanäle hindurch gepumpt, sodass das nicht polymerisierte Hydrogel durch den Spülpuffer verdrängt und durch die jeweils zweite Öffnung aus den Fluidkanälen entfernt wird.

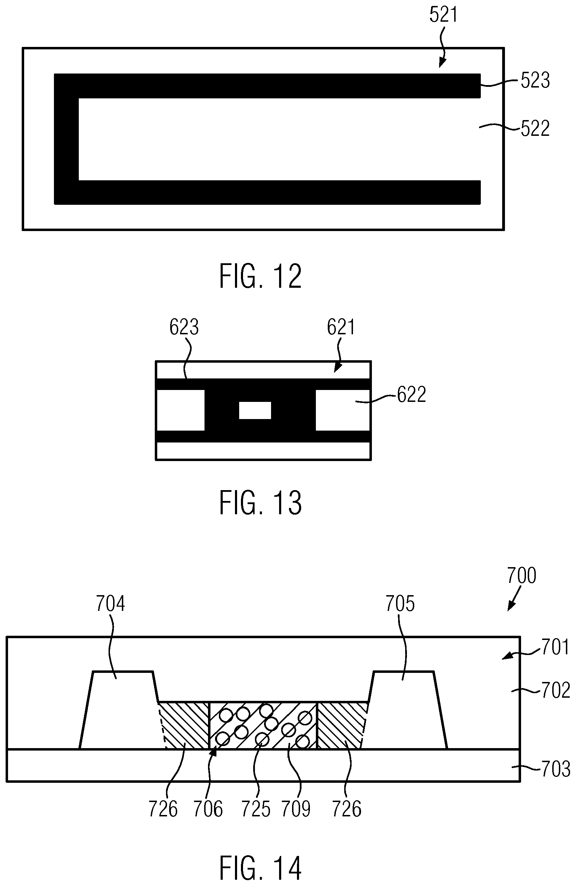

Mittels der oben beschriebenen Schritte kann beispielsweise ein Fluidkanalsystem aus der ersten Ausführungsform hergestellt werden. Auf ähnliche Art und Weise kann auch ein Fluidkanalsystem aus der zweiten Ausführungsform hergestellt werden, wobei hier die Maske 521 beispielsweise die in Figur 12 gezeigte Form hat.

Bei dem oben genannten Verfahren, ist es ebenfalls möglich, dass der Belichtungsschritt ein erster Belichtungsschritt ist, bei dem eine andere Maske 621 als die in Figur 8 gezeigte Maske verwendet wird, wobei die Chromstreifen 623 der Maske nicht nur die Fluidkanäle und sondern auch einen Teil der Kammer verdecken, wie beispielsweise in Figur 13 gezeigt. In diesem Fall ist also die Kammer nach dem Belichtungsschritt und dem Spülen nicht vollständig mit Hydrogel gefüllt. Auf das erste Spülen folgt ein weiterer Schritt, in dem wiederum ein nicht-polymerisiertes Hydrogel durch die Öffnungen nach außen in die Fluidkanäle eingefüllt wird und ein zweiter Belichtungsschritt mit einer zweiten Maske durchgeführt wird. In der zweiten Maske verdecken wiederum Chromstreifen die beiden Fluidkanäle, jedoch nicht die Kammer bzw. nur Teile der Kammer. Diese Verfahrensschritte können so oft wiederholt werden, bis die Kammer zu mindestens 90 % mit Hydrogel gefüllt ist.

Ein solches mehrstufiges Verfahren resultiert beispielsweise in der in Figur 14 im Querschnitt gezeigten Struktur. Dabei können die hintereinander verwendeten Hydrogele verschiedene Eigenschaften haben. Beispielsweise können in dem zuerst eingebrachten Hydrogel 709 Zellen 725 suspensiert sein. In dem zweiten Hydrogel 726 können dagegen keine Zellen suspensiert sein. Das zweite Hydrogel, welches an das Hydrogel angrenzt, kann sich beispielsweise in der Migrierbarkeit für die Zellen oder in der Flüssigkeitsdurchlässigkeit von dem ersten Hydrogel unterscheiden. Beispielsweise kann das zweite Hydrogel für die Zellen im ersten Hydrogel migrierbar sein. An das zweite Hydrogel angrenzend kann (hier nicht gezeigt) ein drittes Hydrogel angeordnet sein, welches eine Migrationsbarriere für Zellen darstellt.

Solche heterogenen Hydrogelstrukturen sind besonders geeignet, um Zellausbreitung und Invasivität zu untersuchen. Beispielsweise kann mit einem Mikroskop beobachtet werden, wie die Zellen in den Invasionsbereich eindringen. Ein Invasionsbereich wird dabei zum Beispiel durch das oben erwähnte dritte Hydrogel begrenzt. So sind die Zellen in einem vorgegebenen Bereich gefangen und stehen zur Analyse, insbesondere zu "Vorher-Nachher-Analysen", zur Verfügung, sodass quantitative Aussagen gemacht werden können. Beispielsweise wird ein Unterschied unmittelbar nach der Präparation und nach einer bestimmten Zeitdauer festgehalten, sodass beispielsweise die Migrationsgeschwindigkeit der Zellen bestimmt werden kann, insbesondere mit oder ohne Zugabe von migrationsfördernden oder migrationsinhibierenden Substanzen.

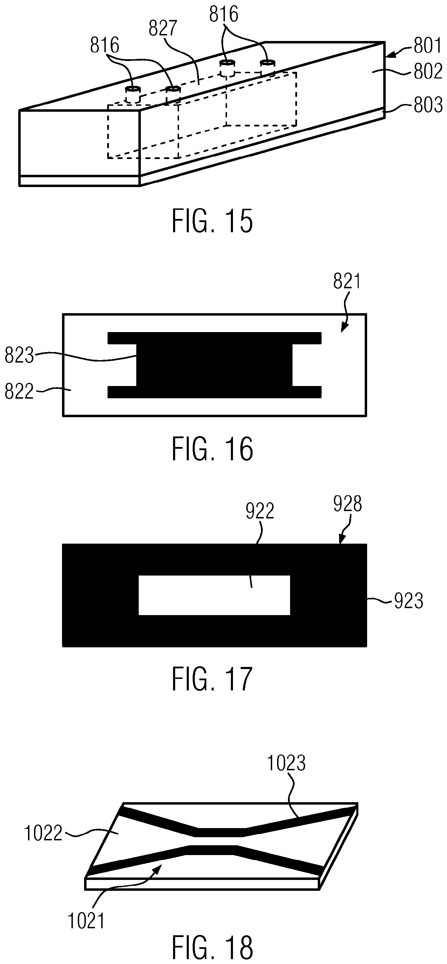

In einem weiteren Ausführungsbeispiel, das in Figur 15 gezeigt ist, für ein Verfahren zum Herstellen eines Fluidkanalsystems wird ein Hohlraum 827, der keine Kanalstrukturen und keine Kammer aufweist, vollständig mit Hydrogel gefüllt. Der Hohlraum wird hier durch ein zweiteiliges Substrat, nämlich durch eine Deckplatte 802, die eine Vertiefung und vier Durchgangslöcher als Öffnungen 816 nach außen umfasst, und durch eine Grundplatte 803 gebildet. Die Grundplatte kann beispielsweise in Form einer Kunststofffolie ausgebildet sein.

In die Öffnungen 816 nach außen wird das flüssige Hydrogel eingefüllt. Anschließend erfolgt, ähnlich wie im vorhergehenden Ausführungsbeispiel, eine Belichtung durch eine Schattenmaske 821. In diesem Beispiel umfasst die Schattenmaske eine Chromschicht 823, die zwei parallel verlaufende Streifen und ein Rechteck, das zwischen den beiden Streifen angeordnet ist, abdeckt. Eine solche Maske ist in Draufsicht in Figur 16 gezeigt.

Erfolgt nun eine Belichtung, beispielsweise wie in dem vorhergehenden Ausführungsbeispiel, wird das Hydrogel in allen Bereichen, außer in dem Bereich der Streifen und des Rechtecks fotopolymerisiert. Das nicht polymerisierte Hydrogel wird durch Spülen entfernt werden. Dabei können entweder Öffnungen nach außen verwendet werden, die bereits in dem Substrat vorhanden waren, oder es können nachträglich Öffnungen nach außen in das Substrat gemacht werden. Auf diese Art und Weise werden in dem Hohlraum zwei Fluidkanäle und eine Kammer ausgebildet, die dann wie in dem ersten Ausführungsbeispiel weiterverarbeitet werden können. Alternativ kann, wie oben beschrieben, der Hohlraum mit flüssigem Hydrogel gefüllt werden und eine wie in Figur 17 gezeigte Maske 928 verwendet werden, bei der eine Glasplatte 922 bis auf einen im Bezug auf ein Substrat mittig angeordneter Bereich, beispielsweise in Form eines Rechtecks, vollständig mit einer Chromschicht 923 versehen ist, sodass nach dem Belichten und Spülen ein Quader von fotopolymerisierten Hydrogel, welches beispielsweise die zusammenhängende Hydrogelstruktur 306 bildet, in der Mitte des Hohlraums stehen bleibt. In einem nächsten Schritt kann der Hohlraum mit einem zweiten Hydrogel gefüllt werden und eine Schattenmaske 421 wie in Figur 8 verwendet werden, sodass das Hydrogel in dem gesamten Hohlraum, bis auf einen Bereich, der später die Fluidkanäle bildet, fotopolymerisiert wird. So erhält man ein wie in Figur 6 dargestelltes Fluidkanalsystem.

Figur 18 zeigt ein Beispiel für eine Maske 1021 umfassend eine Glasplatte 1022 und eine Chromschicht 1023, mit der mit den oben beschriebenen Verfahren abgewinkelte Fluidkanäle hergestellt werden können.

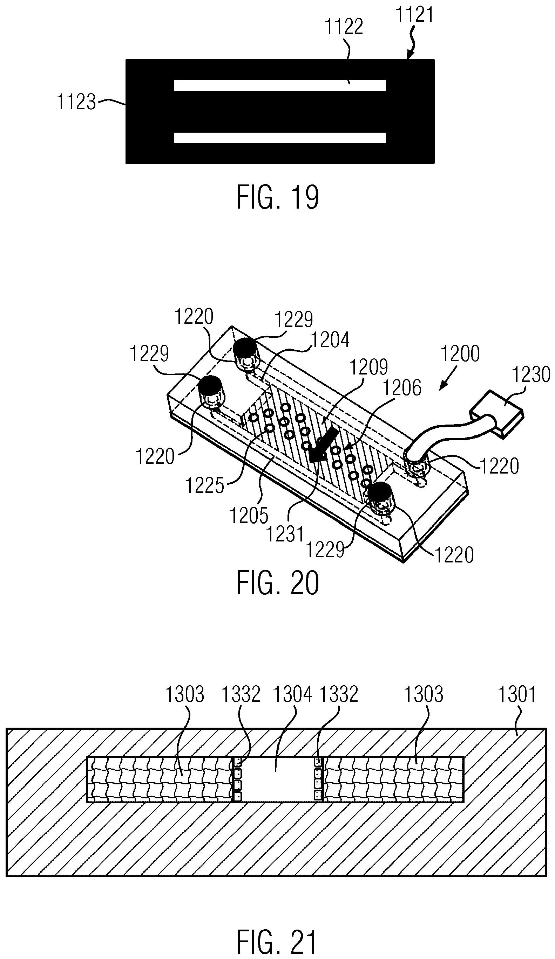

Die oben genannten Verfahren können jeweils auch mit einem fotodepolymerisierbaren Hydrogel durchgeführt werden. Dazu wird die Kammer und der mindestens eine Fluidkanal bzw. der Hohlraum mit dem fotodepolymerisierbaren Hydrogel gefüllt. Dieses wird dann polymerisiert, beispielsweise durch Erwärmung, Abkühlung oder Änderung des PH-Wertes. Eine Maske, die beispielsweise invers zu den oben beschriebenen Masken ist, wird für den Belichtungsschritt verwendet. So wird der belichtete Anteil des polymerisierten Hydrogels wieder depolymerisiert, nämlich das Hydrogel in dem bzw. den Fluidkanälen, und kann wie oben beschrieben weggespült werden. Als Beispiel ist in Figur 19 eine zu der in Figur 8 gezeigten Maske inverse Maske 1121 umfassend eine Glasplatte 1122 mit einer Chromschicht 1123 dargestellt. Beim Belichten wird das Hydrogel im Bereich der Kanäle depolymerisiert. Auch eine zu den jeweiligen Masken in Figuren 12, 13 und 16 bis 18 inverse Maske kann verwendet werden, wenn im Zusammenhang mit dem jeweiligen Verfahren mit fotodepolymerisierbaren Hydrogel gearbeitet wird. Diese Masken sind nicht eigens dargestellt. Dass die Masken invers zu den oben gezeigten Masken sind, ist optional und nur als Beispiel zum besseren Verständnis aufgeführt. Die Maske muss nur gewährleisten, dass ausschließlich ausgewählte Hydrogelbereiche, aus denen das Hydrogel anschließend weggespült werden soll, belichtet werden.

In Figur 20 ist beispielhaft eine Verwendung der Vorrichtung aus Figur 4 schematisch gezeigt. Die Verwendung kann auf ähnliche Weise auch mit anderen Vorrichtungen erfolgen, insbesondere den in den Figuren 5, 6 und 14 gezeigten Vorrichtungen. In dem Hydrogel 1209 in der Kammer 1206 sind Zellen 1225 angeordnet. In die beiden Fluidkanäle 1104, 1105 wird durch eine oder beide Öffnungen 1220 nach außen eine Flüssigkeit eingefüllt. In dem Fluidkanal 1204 befindet sich ein Zellmedium zur Versorgung der Zellen, beispielsweise DMEM. Der Fluidkanal 1205 wird dann an beiden Öffnungen nach außen verschlossen und der Fluidkanal 1104 wird an einer Öffnung verschlossen. Dazu werden beispielsweise Stöpsel 1229 verwendet. An die nicht verschlossene Öffnung des Fluidkanals 1204 wird eine Pumpe 1230 angeschlossen. Mit dieser Pumpe wird ein Druck angelegt, sodass der Druck im Fluidkanal 1204 größer ist als der Druck im Fluidkanal 1205. So wird ein interstitieller Fluss des Zellmediums durch das Hydrogel (angedeutet durch Pfeil 1231) bewirkt.

Die Öffnungen nach außen müssen bei dem Verfahren nicht verschlossen werden. Optional kann auch an den Fluidkanal 1205 eine Pumpe angeschlossen werden. Die jeweilige Flüssigkeit kann dann beispielsweise auch durch die Fluidkanäle gepumpt werden.

Mit einem hier nicht gezeigtem Mikroskop oder anderen Messverfahren, besonders optischen Messverfahren, können die Zellen in dem Hydrogel beobachtet werden, beispielsweise in Transmission. So kann Zellwachstum und Migration untersucht werden.

Bei einer weiteren Verwendung einer erfindungsgemäßen Vorrichtung werden Endothelzellen oder Epithelzellen 1332 als Suspension in einem Puffer in den Fluidkanal 1304 eingefüllt. In diesem Fall ist der Fluidkanal in einem Hydrogel 1303 in dem Substrat 1301 ausgebildet (beispielsweise wie das Fluidkanalsystem, das im Zusammenhang mit Figur 6 beschrieben wurde, und/oder einem Fluidkanalsystem, das mit dem im Zusammenhang mit Figur 15 beschriebenen Verfahren hergestellt wurde). Die Zellen besiedeln, wie in dem Querschnitt in Figur 21 gezeigt, die Hydrogelwände und bilden dadurch eine biologisch relevante Begrenzung zu dem Hydrogel. Dadurch simuliert der Fluidkanal Blutgefäße, mit ähnlichen Barriereeigenschaften wie die biologischen Blutgefäßwände. Es ist ebenfalls möglich, dass der Fluidkanal nicht in einem Hydrogel ausgebildet ist und die Endothelzellen oder Epithelzellen die Wände des Hydrogels in der Kammer besiedeln.

Diese Verwendung kann mit der oben genannten Verwendung kombiniert werden. So kann der Übertritt von Partikeln aus dem Blut in Gewebe nachgestellt und untersucht werden, beispielsweise mittels Mikroskopie.

Es versteht sich, dass in den zuvor beschriebenen Ausführungsbeispielen genannte Merkmale nicht auf diese speziellen Kombinationen beschränkt sind und auch in beliebigen anderen Kombinationen möglich sind.

## Patentansprüche

1. Fluidkanalsystem (100, 200) zur Untersuchung von Zellen, umfassend eine Kammer (106, 206), die mit mindestens einem fotopolymerisierten Hydrogel (109, 209) und/oder einem polymerisierten fotodepolymerisierbaren Hydrogel zu mindestens 90%, insbesondere vollständig, gefüllt ist,
wobei die Kammer (106; 206) über mindestens zwei Öffnungen (107, 108; 207, 208) ausschließlich zu mindestens einem Fluidkanal (104, 105; 204) hin offen ist,
wobei der mindestens eine Fluidkanal (104, 105; 204) und die Kammer (106; 206) jeweils in Form eines Hohlraums in einem Substrat (101; 201) ausgebildet sind, und
wobei jeder Fluidkanal (104, 105; 204) zwei Öffnungen (116, 216) nach außen aufweist, wobei sich "außen" dabei auf einen Bereich außerhalb des Substrats bezieht, und wobei die Kammer (106; 206) keine direkte Verbindung nach außen hat.

2. Fluidkanalsystem (300) zur Untersuchung von Zellen, umfassend einen Hohlraum (827) in einem Substrat (301), wobei in dem Hohlraum (827) mindestens ein fotopolymerisiertes Hydrogel (309) und/oder ein polymerisiertes fotodepolymerisierbares Hydrogel angeordnet ist, wobei das Hydrogel jeweils derart strukturiert ist, dass mindestens ein Fluidkanal (304, 305) und eine zusammenhängende Hydrogelstruktur (306) ausgebildet sind, und
wobei die Hydrogelstruktur (306) an zwei getrennten Oberflächenbereichen (307, 308) an den mindestens einen Fluidkanal (304, 305) angrenzt und ausschließlich über den mindestens einen Fluidkanal (304, 305) eine Verbindung nach außen hat.

3. Fluidkanalsystem nach einem der vorangegangenen Ansprüche, wobei der mindestens eine Fluidkanal (104, 105; 204; 304, 305) genau einen Fluidkanal (204) umfasst, und
wobei, wenn das Fluidkanalsystem das Fluidkanalsystem nach Anspruch 1 ist, die Kammer (206) an einem ersten Teilbereich des Fluidkanals (204) und an einem zweiten Teilbereich des Fluidkanals (204) zu dem Fluidkanal (204) hin offen ist, bzw.
wobei, wenn das Fluidkanalsystem das Fluidkanalsystem nach Anspruch 2 ist, einer der zwei Oberflächenbereiche (307) der Hydrogelstruktur (306) an einen ersten Teilbereich des Fluidkanals (204) angrenzt und der andere der zwei Oberflächenbereiche (308) der Hydrogelstruktur (306) an einen zweiten Teilbereich des Fluidkanals (204) angrenzt.

4. Fluidkanalsystem nach einem der vorangegangenen Ansprüche, wobei der mindestens eine Fluidkanal (104, 105; 204; 304, 305) einen ersten Fluidkanal (104; 304) und einen zweiten Fluidkanal (105; 305) umfasst, und
wobei, wenn das Fluidkanalsystem das Fluidkanalsystem nach Anspruch 1 ist, die mindestens zwei Öffnungen (107, 108) der Kammer (106) eine erste Öffnung (107) zu dem ersten Fluidkanal (104) und eine zweite Öffnung (108) zu dem zweiten Fluidkanal (105) hin umfassen, bzw.
wobei, wenn das Fluidkanalsystem das Fluidkanalsystem nach Anspruch 2 ist, die zwei getrennten Oberflächenbereiche (307, 308) einen ersten Oberflächenbereich (307), der an den ersten Fluidkanal (304) angrenzt, und einen zweiten Oberflächenbereich (308), der an den zweiten Fluidkanal (305) angrenzt, umfassen,
insbesondere wobei die Kammer (106) bzw. die Hydrogelstruktur (306) ganz oder teilweise zwischen dem ersten Fluidkanal (104; 304) und dem zweiten Fluidkanal (105; 305) angeordnet ist.

5. Fluidkanalsystem nach einem der vorangegangenen Ansprüche, wobei das mindestens eine Hydrogel (109; 209) bzw. die Hydrogelstruktur (306) mindestens zwei verschiedene Hydrogele (709, 726) umfasst.

6. Fluidkanalsystem nach einem der vorangegangenen Ansprüche, wobei das bzw. mindestens eines der, insbesondere alle, Hydrogele (109; 209; 409; 709; 726) bzw. die Hydrogelstruktur (306) Zellen (725) umfassen.

7. Fluidkanalsystem nach einem der vorangegangen Ansprüche, wobei das Substrat (101; 201; 301) einteilig oder mehrteilig ausgebildet ist, insbesondere wobei das Substrat (101; 201; 301) eine Glasplatte und/oder eine Kunststoffplatte und/oder eine Kunststofffolie umfasst.

8. Verfahren zum Herstellen eines Fluidkanalsystems (100; 200) nach einem der Ansprüche 1, 3 bis 7, umfassend
Bereitstellen des Substrats (101; 201; 401; 701) mit der Kammer (106; 206; 406; 706) und dem mindestens einen Fluidkanal (104, 105; 204; 404, 405; 704, 705),
Befüllen der Kammer (106; 206; 406; 706) mit fotopolymerisierbarem Hydrogel durch den mindestens einen Fluidkanal (104, 105; 204; 404, 405; 704, 705),
selektives Belichten des Hydrogels, insbesondere unter Verwendung einer Schattenmaske (421; 521; 621), sodass in der Kammer (106; 206; 406; 706) befindliches Hydrogel zumindest teilweise fotopolymerisiert wird, und
Spülen jedes Fluidkanals (104, 105; 204; 404, 405; 704, 705) zum Entfernen von nicht polymerisiertem Hydrogel.

9. Verfahren zum Herstellen eines Fluidkanalsystems (100; 200) nach einem der Ansprüche 1, 3 bis 7, umfassend
Bereitstellen des Substrats (101; 201; 401; 701) mit der Kammer (106; 206; 406; 706) und dem mindestens einen Fluidkanal (104, 105; 204; 404, 405; 704, 705),
Befüllen der Kammer (106; 206; 406; 706) mit fotodepolymerisierbaren Hydrogel durch den mindestens einen Fluidkanal (104, 105; 204; 404, 405; 704, 705),
Polymerisieren des Hydrogels,
selektives Belichten des Hydrogels, insbesondere unter Verwendung einer Schattenmaske (421; 521; 621), sodass in jedem Fluidkanal (104, 105; 204; 404, 405; 704, 705) befindliches Hydrogel fotodepolymerisiert wird, und
Spülen jedes Fluidkanals (104, 105; 204; 404, 405; 704, 705) zum Entfernen von nicht polymerisiertem Hydrogel.

10. Verfahren zum Herstellen eines Fluidkanalsystems (300; 800) nach einem der Ansprüche 2 bis 7, umfassend
Bereitstellen des Substrats (301; 401; 801) mit dem Hohlraum (827),
Befüllen des Hohlraums (827) mit fotopolymerisierbarem Hydrogel,
selektives Belichten des Hydrogels, insbesondere unter Verwendung einer Schattenmaske (821), sodass in dem Hohlraum befindliches Hydrogel zumindest teilweise fotopolymerisiert wird, derart, dass der mindestens eine Fluidkanal (304, 305) und die zusammenhängende Hydrogelstruktur (306) ausgebildet werden, und
Spülen jedes Fluidkanals (304, 305) zum Entfernen von nicht polymerisiertem Hydrogel.

11. Verfahren zum Herstellen eines Fluidkanalsystems (300; 800) nach einem der Ansprüche 2 bis 7, umfassend
Bereitstellen des Substrats (301; 401; 801) mit dem Hohlraum (827),
Befüllen des Hohlraums (827) mit fotodepolymerisierbarem Hydrogel,
Polymerisieren des Hydrogels,
selektives Belichten des Hydrogels, insbesondere unter Verwendung einer Schattenmaske (821), sodass in dem Hohlraum befindliches Hydrogel zumindest teilweise fotodepolymerisiert wird, derart, dass der mindestens eine Fluidkanal (304, 305) und die zusammenhängende Hydrogelstruktur (306) ausgebildet werden, und
Spülen jedes Fluidkanals (304, 305) zum Entfernen von nicht polymerisiertem Hydrogel.

12. Verfahren nach einem der Ansprüche 8 bis 11, wobei das Ausbilden des mindestens einen Fluidkanals (304, 305) ein Ausbilden genau eines oder mindestens zweier, insbesondere genau zweier, Fluidkanäle umfasst.

13. Verfahren nach einem der Ansprüche 8 bis 12, umfassend, vor dem Befüllen, eine vollständige oder teilweise Funktionalisierung der Oberfläche des Substrats (101; 201; 301; 401; 701; 801), insbesondere der Wände der Kammer (106; 206; 406; 706), um das Hydrogel (109; 209; 309; 409; 709, 726), insbesondere chemisch, an die Oberfläche des Substrats (101; 201; 301; 401; 701; 801) zu binden.

14. Verfahren nach einem der Ansprüche 8, 9 und 13, wobei das fotopolymerisierbare Hydrogel bzw. das fotodepolymerisierbare Hydrogel ein erstes fotopolymerisierbares Hydrogel (726) bzw. ein erstes fotodepolymerisierbares Hydrogel ist, wobei die Kammer (106; 206; 406; 706) nach dem Befüllen, Belichten und Spülen des ersten fotopolymerisierbaren Hydrogels bzw. des ersten fotodepolymerisierbaren Hydrogels nicht vollständig mit Hydrogel (109; 209; 709) gefüllt ist, umfassend
Befüllen der Kammer (106; 206; 406; 706) mit einem zweiten Hydrogel durch den mindestens einen Fluidkanal (104, 105; 204; 404, 405; 704, 705), wobei das zweite Hydrogel fotopolymerisierbar oder fotodepolymerisierbar ist,
selektives Belichten des zweiten Hydrogels (726), insbesondere unter Verwendung einer Schattenmaske, sodass in der Kammer (106; 206; 406; 706) befindliches zweites Hydrogel (726) zumindest teilweise fotopolymerisiert wird bzw. zweites Hydrogel in jedem Fluidkanal (104, 105; 204; 404, 405; 704, 705) fotodepolymerisiert wird, und
Spülen jedes Fluidkanals (104, 105; 204; 404, 405; 704, 705) zum Entfernen von nicht polymerisiertem zweitem Hydrogel (726).

15. Verfahren nach einem der Ansprüche 8 bis 14, wobei das bzw. mindestens eines der fotopolymerisierbaren Hydrogele (109; 209; 709; 726) bzw. mindestens eines der fotodepolymerisierbaren Hydrogele beim Befüllen Zellen (725) umfassen.

## Claims

1. Fluid channel system (100, 200) for examining cells, comprising a chamber (106, 206) which is filled with at least one photopolymerized hydrogel (109, 209) and/or one polymerized photodepolymerizable hydrogel to at least 90 % of capacity, in particular completely,
wherein the chamber (106; 206) is open via at least two openings (107, 108; 207, 208) exclusively towards at least one fluid channel (104, 105; 204),
wherein the at least one fluid channel (104, 105; 204) and the chamber (106; 206) are each embodied in the form of a hollow space in a substrate (101; 201), and
wherein each fluid channel (104, 105; 204) has two openings (116, 216) to the outside, wherein "outside" refers to a region outside the substrate, and wherein the chamber (106; 206) does not have a direct connection to the outside.

2. Fluid channel system (300) for examining cells, comprising a hollow space (827) in a substrate (301), wherein in the hollow space (827), at least one photopolymerized hydrogel (309) and/or one polymerized photodepolymerizable hydrogel is arranged, wherein the respective hydrogel is structured such that at least one fluid channel (304, 305) and a coherent hydrogel structure (306) are formed, and
wherein the hydrogel structure (306) adjoins the at least one fluid channel (304, 305) at two separate surface areas (307, 308) and has a connection to the outside exclusively via the at least one fluid channel (304, 305).

3. Fluid channel system according to one of the preceding claims, wherein the at least one fluid channel (104, 105; 204; 304, 305) comprises exactly one fluid channel (204), and
wherein, in case the fluid channel system is the fluid channel system according to claim 1, the chamber (206) is open towards the fluid channel (204) at a first partial area of the fluid channel (204) and a second partial area of the fluid channel (204), or
wherein, in case the fluid channel system is the fluid channel system according to claim 2, one of the two surface areas (307) of the hydrogel structure (306) adjoins a first partial area of the fluid channel (204), and the other one of the two surface areas (308) of the hydrogel structure (306) adjoins a second partial area of the fluid channel (204).

4. Fluid channel system according to one of the preceding claims, wherein the at least one fluid channel (104, 105; 204; 304, 305) comprises a first fluid channel (104; 304) and a second fluid channel (105; 305), and
wherein, in case the fluid channel system is the fluid channel system according to claim 1, the at least two openings (107, 108) of the chamber (106) comprise a first opening (107) to the first fluid channel (104) and a second opening (108) to the second fluid channel (105), or
wherein, in case the fluid channel system is the fluid channel system according to claim 2, the two separate surface areas (307, 308) comprise a first surface area (307) adjoining the first fluid channel (304) and a second surface area (308) adjoining the second fluid channel (305),
in particular wherein the chamber (106) or the hydrogel structure (306) is arranged completely or partially between the first fluid channel (104; 304) and the second fluid channel (105; 305).

5. Fluid channel system according to one of the preceding claims, wherein the at least one hydrogel (109; 209) or the hydrogel structure (306) comprises at least two different hydrogels (709, 726).

6. Fluid channel system according to one of the preceding claims, wherein the or at least one of the, in particular all, hydrogels (109; 209; 409; 709; 726) or the hydrogel structure (306) comprise(s) cells (725).

7. Fluid channel system according to one of the preceding claims, wherein the substrate (101; 201; 301) has a one-piece or multi-piece configuration, in particular wherein the substrate (101; 201; 301) comprises a glass plate and/or a plastic plate and/or a plastic foil.

8. Method of manufacturing a fluid channel system (100; 200) according to one of claims 1, 3 to 7, comprising
providing the substrate (101; 201; 401; 701) with the chamber (106; 206; 406; 706) and the at least one fluid channel (104, 105; 204; 404, 405; 704, 705),
filling the chamber (106; 206; 406; 706) with photopolymerizable hydrogel through the at least one fluid channel (104, 105; 204; 404, 405; 704, 705),
selectively exposing the hydrogel, in particular using a shadow mask (421; 521; 621), so that hydrogel located in the chamber (106; 206; 406; 706) is at least partially photopolymerized, and
flushing each fluid channel (104, 105; 204; 404, 405; 704, 705) for removing non-polymerized hydrogel.

9. Method of manufacturing a fluid channel system (100; 200) according to one of claims 1, 3 to 7, comprising
providing the substrate (101; 201; 401; 701) with the chamber (106; 206; 406; 706) and the at least one fluid channel (104, 105; 204; 404, 405; 704, 705),
filling the chamber (106; 206; 406; 706) with photodepolymerizable hydrogel through the at least one fluid channel (104, 105; 204; 404, 405; 704, 705),
polymerizing said hydrogel,
selectively exposing said hydrogel, in particular using a shadow mask (421; 521; 621), so that hydrogel located in each fluid channel (104, 105; 204; 404, 405; 704, 705) is photodepolymerized, and
flushing each fluid channel (104, 105; 204; 404, 405; 704, 705) for removing non-polymerized hydrogel.

10. Method of manufacturing a fluid channel system (300; 800) according to one of claims 2 to 7, comprising
providing the substrate (301; 401; 801) with said hollow space (827),
filling the hollow space (827) with photopolymerizable hydrogel,
selectively exposing said hydrogel, in particular using a shadow mask (821), so that hydrogel located in the hollow space is at least partially photopolymerized, such that the at least one fluid channel (304, 305) and the coherent hydrogel structure (306) are formed, and
flushing each fluid channel (304, 305) for removing non-polymerized hydrogel.

11. Method of manufacturing a fluid channel system (300; 800) according to one of claims 2 to 7, comprising
providing the substrate (301; 401; 801) with said hollow space (827),
filling said hollow space (827) with photodepolymerizable hydrogel,
polymerizing said hydrogel,
selectively exposing said hydrogel, in particular using a shadow mask (821), so that hydrogel located in the hollow space is at least partially photodepolymerized, such that the at least one fluid channel (304, 305) and the coherent hydrogel structure (306) are formed, and
flushing each fluid channel (304, 305) for removing non-polymerized hydrogel.

12. Method according to one of claims 8 to 11, wherein the formation of said at least one fluid channel (304, 305) comprises the formation of exactly one or at least two, in particular exactly two fluid channels.

13. Method according to one of claims 8 to 12, comprising, before the filling, a complete or partial functionalization of the surface of the substrate (101; 201; 301; 401; 701; 801), in particular of the walls of the chamber (106; 206; 406; 706), to bind the hydrogel (109; 209; 309; 409; 709, 726), in particular chemically, to the surface of the substrate (101; 201; 301; 401; 701; 801).

14. Method according to one of claims 8, 9 and 13, wherein the photopolymerizable hydrogel or the photodepolymerizable hydrogel is a first photopolymerizable hydrogel (726) or a first photodepolymerizable hydrogel, wherein the chamber (106; 206; 406; 706) is, after the filling, exposing and flushing the first photopolymerizable hydrogel or the first photodepolymerizable hydrogel, not completely filled with hydrogel (109; 209; 709), comprising
filling the chamber (106; 206; 406; 706) with a second hydrogel through the at least one fluid channel (104, 105; 204; 404, 405; 704, 705), wherein the second hydrogel is photopolymerizable or photodepolymerizable,
selectively exposing said second hydrogel (726), in particular using a shadow mask, so that second hydrogel (726) located in the chamber (106; 206; 406; 706) is at least partially photopolymerized or second hydrogel in each fluid channel (104, 105; 204; 404, 405; 704, 705) is photodepolymerized, and
flushing each fluid channel (104, 105; 204; 404, 405; 704, 705) for removing non-polymerized second hydrogel (726).

15. Method according to one of claims 8 to 14, wherein the or at least one of the photopolymerizable hydrogels (109; 209; 709; 726) or at least one of the photodepolymerizable hydrogels comprises cells (725) during filling.

## Revendications

1. Système à canal de fluide (100, 200) pour l'examen de cellules, comprenant une chambre (106, 206), qui est remplie à au moins 90%, notamment en totalité, par au moins un hydrogel photopolymérisé (109, 209) et/ou un hydrogel photopolymérisé pouvant être photo-dépolymérisé,
système
dans lequel la chambre (106; 206) est ouverte exclusivement vers au moins un canal de fluide (104, 105; 204) par l'intermédiaire d'au moins deux ouvertures (107, 108; 207, 208),
dans lequel ledit au moins un canal de fluide (104, 105; 204) et la chambre (106; 206) sont réalisés respectivement sous la forme d'une cavité dans un substrat (101; 201), et
dans lequel chaque canal de fluide (104, 105; 204) présente deux ouvertures (116, 216) vers l'extérieur, "l'extérieur" se rapportant ici à une zone en-dehors du substrat, et la chambre (106; 206) n'ayant pas de liaison directe vers l'extérieur.

2. Système à canal de fluide (300) pour l'examen de cellules, comprenant une cavité (827) dans un substrat (301),
système
dans lequel à l'intérieur de la cavité (827) est agencé au moins un hydrogel photopolymérisé (309) et/ou un hydrogel photopolymérisé pouvant être photo-dépolymérisé,
dans lequel l'hydrogel est respectivement structuré de manière telle que soient réalisés au moins un canal de fluide (304, 305) et une structure d'hydrogel (306) continue, et
dans lequel la structure d'hydrogel (306) est adjacente au dit au moins un canal de fluide (304, 305) au niveau de deux zones de surface (307, 308) séparées, et présente une liaison vers l'extérieur exclusivement par l'intermédiaire dudit au moins un canal de fluide (304, 305) .

3. Système à canal de fluide selon l'une des revendications précédentes,
dans lequel ledit au moins un canal de fluide (104, 105; 204; 304, 305) comprend exactement un canal de fluide (204), et
dans lequel, lorsque le système à canal de fluide est le système à canal de fluide selon la revendication 1, la chambre (206) est ouverte vers le canal de fluide (204) au niveau d'une première zone partielle du canal de fluide (204) et au niveau d'une deuxième zone partielle du canal de fluide (204), ou respectivement
dans lequel, lorsque le système à canal de fluide est le système à canal de fluide selon la revendication 2, l'une des deux zones de surface (307) de la structure d'hydrogel (306) est adjacente à une première zone partielle du canal de fluide (204), et l'autre des deux zones de surface (308) de la structure d'hydrogel (306) est adjacente à une deuxième zone partielle du canal de fluide (204).

4. Système à canal de fluide selon l'une des revendications précédentes,
dans lequel ledit au moins un canal de fluide (104, 105; 204; 304, 305) comprend un premier canal de fluide (104; 304) et un deuxième canal de fluide (105; 305), et
dans lequel, lorsque le système à canal de fluide est le système à canal de fluide selon la revendication 1, lesdites au moins deux ouvertures (107, 108) de la chambre (106) comprennent une première ouverture (17) vers le premier canal de fluide (104), et une deuxième ouverture (108) vers le deuxième canal de fluide (105), ou respectivement
dans lequel, lorsque le système à canal de fluide est le système à canal de fluide selon la revendication 2, les deux zones de surface (307, 308) séparées comprennent une première zone de surface (307), qui est adjacente au premier canal de fluide (304), et une deuxième zone de surface (308), qui est adjacente au deuxième canal de fluide (305),
notamment dans lequel la chambre (106), respectivement la structure d'hydrogel (306), est agencée en totalité ou en partie entre le premier canal de fluide (104; 304) et le deuxième canal de fluide (105; 305).

5. Système à canal de fluide selon l'une des revendications précédentes, dans lequel ledit au moins un hydrogel (109; 209) ou la structure d'hydrogel (306) comprend au moins deux hydrogels différents (709, 726).

6. Système à canal de fluide selon l'une des revendications précédentes, dans lequel l'hydrogel ou respectivement au moins l'un des hydrogels, notamment tous les hydrogels (109; 209; 409; 709; 726), ou respectivement la structure d'hydrogel (306), comprennent des cellules (725).

7. Système à canal de fluide selon l'une des revendications précédentes, dans lequel le substrat (101; 201; 301) est réalisé en une seule partie ou en plusieurs parties, notamment dans lequel le substrat (101; 201; 301) comprend une plaque de verre et/ou une plaque de matière plastique et/ou une feuille de matière plastique.

8. Procédé pour fabriquer un système à canal de fluide (100; 200) selon l'une des revendications 1, 3 à 7, comprenant
la fourniture et la préparation du substrat (101; 201; 401; 701) avec la chambre (106; 206; 406; 706) et ledit au moins un canal de fluide (104, 105; 204; 404, 405; 704, 705),
le remplissage de la chambre (106; 206; 406; 706) avec un hydrogel susceptible d'être photopolymérisé, à travers ledit au moins un canal de fluide (104, 105; 204; 404, 405; 704, 705),
l'irradiation lumineuse sélective de l'hydrogel, notamment en utilisant un masque d'ombrage (421; 521; 621), de sorte que de l'hydrogel se trouvant dans la chambre (106; 206; 406; 706) va être au moins partiellement photopolymérisé, et
le rinçage de chaque canal de fluide (104, 105; 204; 404, 405; 704, 705) pour éliminer de l'hydrogel non polymérisé.

9. Procédé pour fabriquer un système à canal de fluide (100; 200) selon l'une des revendications 1, 3 à 7, comprenant
la fourniture et la préparation du substrat (101; 201; 401; 701) avec la chambre (106; 206; 406; 706) et ledit au moins un canal de fluide (104, 105; 204; 404, 405; 704, 705),
le remplissage de la chambre (106; 206; 406; 706) avec un hydrogel susceptible d'être photo-dépolymérisé, à travers ledit au moins un canal de fluide (104, 105; 204; 404, 405; 704, 705),
la polymérisation de l'hydrogel,
l'irradiation lumineuse sélective de l'hydrogel, notamment en utilisant un masque d'ombrage (421; 521; 621), de sorte que de l'hydrogel se trouvant dans chaque canal de fluide (104, 105; 204; 404, 405; 704, 705) va être photo-dépolymérisé, et
le rinçage de chaque canal de fluide (104, 105; 204; 404, 405; 704, 705) pour éliminer de l'hydrogel non polymérisé.

10. Procédé pour fabriquer un système à canal de fluide (300; 800) selon l'une des revendications 2 à 7, comprenant
la fourniture et la préparation du substrat (301; 401; 801) avec la cavité (827),
le remplissage de la cavité (827) avec un hydrogel susceptible d'être photopolymérisé,
l'irradiation lumineuse sélective de l'hydrogel, notamment en utilisant un masque d'ombrage (821), de sorte que de l'hydrogel se trouvant dans la cavité va être au moins partiellement photopolymérisé, de manière à former ledit au moins un canal de fluide (304, 305) et la structure d'hydrogel (306) continue, et
le rinçage de chaque canal de fluide (304, 305) pour éliminer de l'hydrogel non polymérisé.

11. Procédé pour fabriquer un système à canal de fluide (300; 800) selon l'une des revendications 2 à 7, comprenant
la fourniture et la préparation du substrat (301; 401; 801) avec la cavité (827),
le remplissage de la cavité (827) avec un hydrogel susceptible d'être photo-dépolymérisé,
la polymérisation de l'hydrogel,
l'irradiation lumineuse sélective de l'hydrogel, notamment en utilisant un masque d'ombrage (821), de sorte que de l'hydrogel se trouvant dans la cavité va être au moins partiellement photo-dépolymérisé, de manière à former ledit au moins un canal de fluide (304, 305) et la structure d'hydrogel (306) continue, et
le rinçage de chaque canal de fluide (304, 305) pour éliminer de l'hydrogel non polymérisé.

12. Procédé selon l'une des revendications 8 à 11, d'après lequel la formation dudit au moins un canal de fluide (304, 305) comprend la formation d'exactement un canal de fluide, ou au moins de deux et notamment exactement de deux canaux de fluide.

13. Procédé selon l'une des revendications 8 à 12, comprenant, avant le remplissage, une fonctionnalisation totale ou partielle de la surface du substrat (101; 201; 301; 401; 701; 801), notamment des parois de la chambre (106; 206; 406; 706), en vue de lier l'hydrogel (109; 209; 309; 409; 709, 726), notamment de manière chimique, à la surface du substrat (101; 201; 301; 401; 701; 801).

14. Procédé selon l'une des revendications 8, 9 et 13, d'après lequel l'hydrogel pouvant être photopolymérisé ou respectivement l'hydrogel pouvant être photo-dépolymérisé est un premier hydrogel (726) pouvant être photopolymérisé et respectivement un premier hydrogel pouvant être photo-dépolymérisé, la chambre (106; 206; 406; 706), après le remplissage, l'irradiation lumineuse et le rinçage du premier hydrogel pouvant être photopolymérisé et respectivement du premier hydrogel pouvant être photo-dépolymérisé, n'étant pas totalement remplie d'hydrogel (109; 209; 709), le procédé comprenant
le remplissage de la chambre (106; 206; 406; 706) avec un deuxième hydrogel à travers ledit au moins un canal de fluide (104, 105; 204; 404, 405; 704, 705), le deuxième hydrogel étant du type pouvant être photopolymérisé ou photo-dépolymérisé,
l'irradiation lumineuse sélective du deuxième hydrogel (726), notamment en utilisant un masque d'ombrage, de sorte que le deuxième hydrogel (726) se trouvant dans la chambre (106; 206; 406; 706) va être au moins partiellement photopolymérisé, et que, respectivement, le deuxième hydrogel va être photo-dépolymérisé dans chaque canal de fluide (104, 105; 204; 404, 405; 704, 705), et
le rinçage de chaque canal de fluide (104, 105; 204; 404, 405; 704, 705) pour éliminer du deuxième hydrogel (726) non polymérisé.

15. Procédé selon l'une des revendications 8 à 14, d'après lequel l'hydrogel, ou au moins l'un des hydrogels (109; 209; 709; 726) pouvant être photopolymérisés, ou respectivement au moins l'un des hydrogels pouvant être photo-dépolymérisés, comportent des cellules (725) lors du remplissage.
